# EUROPEAN PATENT APPLICATION

(11) **EP 2 599 859 A1**
(43) Date of publication of application: **05.06.2013**
(21) Application number: 11191408.1
(22) Date of filing: 30.11.2011
(51) Int. Cl.: C12N 5/0735

(54) **Haploid cells**

(71) Applicant: IMBA-Institut für Molekulare Biotechnologie GmbH, 1030 Wien (AT)
(72) Inventor: Elling, Ulrich, 1040 Vienna (AT); Penninger, Josef, 1130 Vienna (AT)
(74) Representative: Sonn & Partner Patentanwälte

(57) **Abstract**

The present invention relates to the generation of stable haploid cell cultures, uses of said cells in forward and reverse genetics, especially the identification of target genes associated with a modified phenotype and in particular identifying genetic targets associated with toxin resistance, especially ricin toxicity resistance, and therapeutic uses of target compounds.

## Description

The present invention relates to haploid cell and their uses as a genetic screening tool as well as newly identified genes associated with cell survival and toxin resistance.

All somatic mammalian cells carry two copies of chromosomes (diploidy), whereas organisms with a single copy of their genome such as yeast provide a basis for recessive genetics. Some organisms such as yeast or social insects are haploid, i.e. they carry a single set of chromosomes. Haploidy in yeast has been utilized to identify fundamental mechanisms of biology. However, all somatic mammalian cells carry two copies of chromosomes (diploidy) which obscure mutational screens, whereas organism with a single copy of their genome such as yeast provide a basis for genetic analyses where any recessive mutation of essential genes will show a clear phenotype due to the absence of a second gene copy. Haploidy has been achieved in fish embryonic stem cells, human KBM-7 leukemia cells, or by electrofusion to generate hybrid cells. However, since haploidy is incompatible with mammalian development, no somatic haploid cell has ever been reported in mammals.

The WO 2008/033469 A1 describes methods of producing parthenogenetic embryonic stem cell with a diploid, heterozygous genome. These cells are matched to a certain donor and can be used for transplants.

The WO 01/32015 A1 relates to a method of isolating haploid cells, especially cells that are produced from diploid oocytes (e.g. after fertilization or introduction of a female pronucleus) in which half the chromosomes are then extruded in the polar body. Such haploid cells are converted to diploid cells, e.g. by electrofusion or injection of another haploid genome, to form a diploid embryo.

Kaufman et al. (1983) describe a method of activating murine oocytes that develop into a mixture of haploid and diploid stem cells. However the haploid cells could not be stably cultivated and cultures immediately converted into an all diploid state.

It is a goal of the present invention to provide a culture of haploid stem cells and methods for generating such cells. Such cell should be stable for a sufficient amount of time that allows manipulation and analysis during forward and reverse genetics experiments.

These goals are solved by the invention, which provides a method of generating a mammal cell or cell line of haploid cells comprising obtaining a plurality of haploid cells in an multicellular embryonic stage of development, preferably a blastocyst stage, isolating cells of said plurality, expanding isolated cells of said plurality, selecting and isolating one or more single cells with a haploid genome from said expanded cells, thereby obtaining a cell of a cell line of stable haploid cells. Optionally several isolated cells can be combined to a cell culture. Alternatively, each individual cell can be proliferated and grown into a cell culture. Also provided is a cell culture of cells with a haploid genome obtainable or obtained by the inventive method and its preferred variants. Further aspects of the invention are described and defined in the claims.

A plurality of haploid cells in a multicellular embryonic stage of development can be obtained from activated oocytes that can be parthenogenically proliferated in a haploid state. In a preferred embodiment the inventive method comprises the steps of obtaining a haploid parthenogenic oozyte or haploid embryonic cell of the mammal, transferring said oozyte or embryonic cell into a pseudopregnant female, allowing said oozyte or embryonic cell to grow to a multicellular stage, optionally culturing cells of said multicellular stage to a blastocyst stage, thereby providing said plurality of haploid cells in an multicellular embryonic stage of development. In further steps the method can comprise obtaining a cell of said multicellular stage, expanding said cells of multicellular stage (simple maintenance e.g. as a culture, especially in isolated form, without expansion is also possible), selecting and isolating a single cell with a haploid genome from said expanded cells, expanding said cell to a cell culture thereby obtaining a cell line of stable haploid cells.

The first step of the method comprises obtaining a haploid parthenogenic oozyte or embryonic cell. This can be done by common methods known in the art, e.g. by activating an oocyte by alcohol or strontium treatment. The activated cells are capable of proliferation and contain only one genome copy (haploidy).

Haploidy relates to the presence of only one genome copy (1n) per cell during the G1 or G0 phase of the cell cycle. The inventive haploid cells are able to replicate the genome in the S phase, establishing a second copy of the genome (2n) in the G2 phase. This copied genome in the G2 phase is homozygous. Haploidy can be distinguished from diploidy, with two genomes (usually heterozygous) during the G1 or G0 phase and four genomes during the G2 phase after the S phase. All inventive cells that eventually lead to the established haploid cell culture maintain haploidy during all steps of the method. Some cell may obtain a diploid genome. These cells are selectively removed.

The activated oocytes can be grown in a pseudopregnant female, wherein said cells are grown to a multicellular stage, such as a blastula or morula stage. The cells of the multicellular stage can be isolated and also further grown in vivo or in vitro. In said further growing step the plurality of cells of said multicellular stage can further expand and/or develop, including to a blastula stage. Although haploid cells cannot complete embryonic development while maintaining haploidy, early stages of development are possible. According to the invention such embryo-like multicellular aggregations can be a plurality of haploid cells in an embryonic stage of development. In such a stage the cells are attached by intercellular bonds within said plurality. Such a plurality can be generated de novo or provided, e.g. from a frozen supply. Alternatively it is also possible to simply obtain an isolated cell from such a plurality or multicellular stage, such as a haploid parthenogenic embryo-like stage.

In a next step one or more cell from said plurality of cells in a multicellular stage is isolated and expanded. Isolation of a cell from a multicellular stage usually involves separating the multicellular stage into individual cells, i.e. individualization, e.g. by disrupting cell adhesion such as by trypsinization, or removing at least one haploid cell from the multicellular stage. The isolated cell is then further expanded or maintained, e.g. in a cell culture medium. From the expanded or maintained cells the inventive haploid cell is selected and isolated. It appears that this additional isolation step is crucial when attempting to obtain a cell culture of haploid cells. Although such a cell is most likely present from the beginning of the parthenogenic activation, the presence of other cells obtained from the blastula stage seem to mask the unique properties of a few selected cells that are present in the plurality of cells in a multicellular stage. Only with this additional isolation step does it seem possible that these selected few cells can maintain their haploid state for a prolonged time.

The isolated cells are single cells without attachment to any other cell of said original plurality of cells of multicellular (embryonic) stage. The cells can be maintained and/or expanded on common feeder cultures, preferably on a layer of feeder cells, such as fibroblasts, preferably mouse embryonic fibroblasts (MEF), especially with inactivated cell cycle e.g. by radiation or mitomycin C. The cells may also be maintained or grown in feeder cell free culture conditions. Preferably the cells are maintained on a feeder layer and then maintained in a feeder cell free culture. During the maintenance of the cells, the cells may also be expanded, proliferated and/or grown.

"Isolation" of a cell as used herein refers to the removal of said cell from other cells of other properties. These other properties may not lie in the genetic make-up of the cells but also in state of activation, differentiation, or expression that may be linked with gene silencing or activation events. In most cases isolation relates to the individualization of single cells but may also relate to isolation of same property or activation status.

Suitable media for stem cell maintenance are known in the art and commercially available; examples are media containing Dulbecco's phosphate buffered saline, MgCl₂, CaCl₂, L glutamine, non-essential amino acids, antibiotics like penicillin/streptomycin (P/S), Fetal bovine serum (FBS), LIF (Leukemia Inhibitory Factor). 0.25% (w/v) trypsin-EDTA may optionally additionally be used to split cells. Additional factors contained in the medium may be human transferrin, putrescine dihydrochloride, human recombinant insulin, L thyroxine, tri-iodothyronine, progesterone, sodium selenite, heparin, and corticosterone.

The initial expansion, after isolation of the cells from a multicellular stage, may be between 10 min to 1 week, preferably, at least 15 min, 20 min or 25 min, and/or up to any one of 5 days, 4 days, 3 days 2 days, 1 days, 18 hours, 12 hours, 6 hours, 3 hours, 1 hour. From the expanded cells single haploid cells are isolated, that can form the basis of a new relatively stable haploid cell culture. These haploid cells have stem cell like characteristics and are referred therein as "stem cells", although of course the development of a haploid mammal or differentiated tissues of haploid cells is not possible. A "stem cell" is a cell that has the ability to proliferate in culture, producing some daughter cells that remain relatively undifferentiated, and other daughter cells that give rise to cells of one or more specialized cell types. The inventive haploid cells are capable of proliferating indefinitely and can differentiate into the three embryonic germ layers. They cannot differentiate into all somatic cell lineages, and the germ line as embryonic stem cells can. However it is possible to transform the haploid cells into diploid cells which are capable of such diverse differentiation capabilities.

If all these steps are followed, the inventive method of generating mammal cells or a cell line of haploid cells comprise the steps of obtaining a haploid parthenogenic oozyte or embryonic cell of said mammal, transferring said oozyte or embryonic cell into a pseudopregnant female, allowing said oozyte or embryonic cell to grow to a multicellular stage, preferably morula or blastula stage, optionally culturing cell of said multicellular stage to a blastocyst stage, obtaining a cell of said multicellular stage, expanding said cell of multicellular stage, selecting and isolating one or more single cells with a haploid genome from said expanded cells, thereby obtaining a cell of a cell line of stably haploid cells.

For selection and isolation haploid cells may be labelled, in particular it is preferred to label the genome, chromosomes or DNA in the cells. Preferably haploid cells are fluorescence labelled. To select and isolate single cells may be separated or individualized, e.g. by trypsinization. Individual cells may then be sorted according to their genome content. In special cases, with the method in accordance with the invention the cells are selected and isolated with a high throughput of at least 20, preferably at least 50, more particularly at least 100 and especially preferably 200, cells per second. High throughput methods have the advantage that a large number of cells can be isolated per unit of time. One such method is, for example, flow cytometry or FACS, with which up to 1000 cells per second or more can be categorized and isolated. It is also possible to label the cells according to another marker, e.g. a marker of an embryonic cell, to further distinguish and select the cells. Preferably this additional selection is done concurrently with the selection of haploid cells, such as by using a further fluorescence dye labelling that additional marker ("multicolour"-based method). The cells may further be selected according to size.

As shown herein and in the examples, following these method steps it as possible to generate haploid mouse stem cell lines from parthenogenetic embryos. These cells carry 20 chromosomes, express stem cell markers, and can develop into all germ-layers (endoderm, mesoderm, ectoderm) in vitro and in vivo.

The inventive method comprises culturing an isolated cell or several isolated cells that have been expanded from the multicellular stage or plurality into a cell culture. Especially the method comprises a regular reseeding of the cell culture or enrichment of haploid cells or removal of diploid cells to maintain haploid integrity of the cell culture. Preferably the method further comprises the steps of culturing said cells of the cell culture and isolating one or more haploid cells from said cell culture after up to 50 passages and expanding said isolated haploid cells as new continued cell culture.

The invention provides the first relatively stable culture of a mammal cell. Preferably the parental and/or the cells of the cell culture are from a mammal that is preferably non-human. Thus especially preferred, for ethical reasons, the cell is a non-human cell. The cell may be murine, primate, porcine, bovine, caprine, equine, ovine, canine, feline, rabbit.

The parental cell can be heterozygous or homozygous, preferably heterozygous. A heterozygous cell contains two different genomes that can give rise to an individual haploid genome in the inventive cell line. Each generation event of a haploid cell from a heterozygous cell usually gives rise to a different combination of the parental genomes and thus a different haploid cell. Preferably the inventive cells of the cell culture are clones, comprising identical haploid genomes. It is also possible that the parental cells are heterozygous, i.e. comprise two identical genomes. All haploid cells from such parental cells are usually identical. The invention is of particular advantage for heterozygous parental cells, since it is possible with the inventive methods to generate homozygous progeny cells via the haploid stem cell route.

The inventive haploid cells may be converted to diploid cells by e.g. via failed cytokinesis and/or endoreplication of the genome or fusion of at least two haploid cells, or by other methods known in the art including electrofusion or injection of another haploid genome (such as in WO 01/32015 A1). Such diploid cells are still homozygous and capable of further embryonic or tissue development - even for stages later than germ layer development, including differentiation into tissue cells. If other genomes than that of the haploid cells are artificially introduced, the diploid cells may also be generated heterozygous. Thus the inventive method may further comprise converting a cell of the inventive culture into a diploid cell and optionally further differentiating said cell, e.g. into a somatic stem cell, or a pluripotent or omnipotent stem cell, such as a interior stomach lining, gastrointestinal tract, lung, muscle, bone, blood, urogenital, epidermal or nervous system cell. In particular the cells may be differentiated to hematopoietic stem cells giving rise to blood cells; bone marrow stromal cells (mesenchymal stem cells) that give rise to e.g. bone cells (osteocytes) and cartilage cells (chondrocytes); multipotent peripheral blood stem cells (PBSCs); adult bone marrow stem cells with the potential to give rise to hepatocytes, cardiomyocytes, neural cells and muscle cells; neural stem cells in the brain giving rise to nerve cells (neurons) as well as non-neuronal cells (astrocytes and oligodendrocytes); epithelial stem cells giving rise to e.g. absorptive cells, goblet cells, Paneth cells, and enteroendocrine cells; skin stem cells (epidermal stem cells giving rise to keratinocytes and follicular stem cells giving rise to the hair follicle); hepatic stem cells giving rise to pancreatic endocrine hormone-producing cells; pancreatic stem and progenitor cells, giving rise to islet cells; stem cells and progenitor cells of the eye (corneal and retinal stem cells); mesoangioblasts (vessel-associated stem cells). By way of example, bone marrow cells and cord blood stem cells are therapeutically useful for blood disorders such as leukemia, multiple myeloma and lymphoma. Stem cells from bone marrow and peripheral blood may be injected either into the coronary arteries or directly into the myocardium for treating severe ischaemic heart disease, transplantable cells including mesenchymal stem cells from bone marrow and CD34+ cells from peripheral blood. Therapeutic benefit may be increased vascularization of myocardium, and formation of new myocardial cells.

The inventive cultures of haploid cells are relatively stable but single cell conversions to a diploid state may occur. It has been shown that the inventive cells are stable for 70 passages. In some cases 2-3% of cell may convert to diploid cells per day. Preferably the inventive cell cultures are regularly purified to remove diploid cells or reseeded. In preferred embodiments the inventive cell cultures comprise or consist of at least 30% haploid cells, preferably at least 40%, at least 50%, at least 60%, at least 70%, at least 80% or at least 90%, haploid cells. Newly seeded cultures or frozen cultures may comprise or consist of 100% haploid cells.

In preferred embodiments the rate of conversion to diploid cells is at most 10% per day, preferably at most 8% per day, at most 6% per day, at most 5% per day, at most 4% per day or at most 3% per day, especially when averaged over 5 consecutive days.

A cell culture of the invention usually contains a plurality of haploid stem cells, preferably at least 10, especially preferred at least 50, more preferred at least 100, at least 500, at least 1000, at least 5000, at least 10000 at least 50000 or even at least 100000 cells, of which cells with the above percentages, e.g. at least 30%, are haploid.

The inventive haploid stem cells were genetically analysed and several characteristic genes with high discriminatory value in gene expression have been identified by quantitative PCR. In the inventive cells, preferably gene Nanog and/or Sall4 are upregulated as compared to IB 10/C cells. Preferably the upregulation is at least 1.2 times, preferably at least 1.5 times. For Nanog the upregulation may also be at least 2 times.

The inventive haploid cells are further capable of embryoid body (EB) formation. Such EB cells may express the endodermal marker Gata4. Differentiation was further confirmed using real time PCR. The expression of prototypic ES cell markers Nanog, Rex1, Oct4, Sox2, Klf4, Sall4, or Kfl2 or any combination thereof may be downregulated in EBs while mRNA expression of the lineage commitment markers Hand1 (mesoderm, trophectoderm), Nkx2-5 and Brachyury (both mesoderm), Nestin (neural), Gata4, Gata6, Foxa2 (all early endoderm), Sox17 (endoderm and mesoderm), Cxcr4R (endoderm), Keratin18 (ectoderm) or any combination thereof may be upregulated relative to the parental haploid stem cells without EB differentiation. These differences also indicate that the inventive haploid stem cells are capable of differentiation into several lineages of all three germ-layers.

Preferably the inventive haploid cells express at least one stem cell marker, such as Oct4 and/or Sox2. Preferably the haploid cells of the invention are able to form EBs that contain Gata4⁺ endodermal cells and/or Tuj1⁺ neurons.

The inventive haploid cells open the possibility to combine the power of a haploid genome with pluripotency of stem cells to uncover fundamental biological processes in defined cell types at a genomic scale. The inventive cells can therefore be used in mutation analysis, including forward and reverse genetics.

For example, the invention provides the use of a cell with a haploid genome for mutation analysis, comprising introducing a mutation into a genetic locus of interest and observing modified activity of the cell related to said genetic locus. The cells with a haploid genome is preferably a mammal cell, especially with stem cell like properties as described above (such as capability to differentiate into EBs), in particular a cell as obtained or obtainable by the inventive method. Due to the haploid nature of the inventive cell recessive mutations can be investigated with increased efficiency due to the absence of other copies of a single gene in other chromosomes. Thus preferably the inventive cells are used to investigate recessive and/or single copy genes in the inventive haploid cells.

Also for this and further uses of the inventive cells, cells may be selected that are stable in haploid form for at least 10 passages, preferably a cell obtainable or obtained by the method according to the present invention. After e.g. 10 passages there should still be at least 40%, at least 50%, at least 60%, at least 70%, at least 75% of the initial haploid cells, especially with the introduced mutation at the genetic locus of interest. Such a genetic locus of interest may e.g. be Drosha or Rarg.

A reversible mutagenesis protocol was developed that allows saturated genetic recessive screens and results in homozygous alleles. This system allowed to generate the first knock-out cell line for the microRNA processing enzyme Drosha.

Thus the invention further provides a cell of a cell culture of haploid cells of the invention, wherein one or more genes of interest have been inactivated. Preferably the cell is of a cell culture wherein all cells have been similarly modified.

Further provided is a haploid mammal cell comprising a knock-out of the gene Drosha and/or Rarg. Said cell is preferably of a stem cell-like phenotype as described above and is e.g. capable to differentiate into EBs. Said cell is preferably of a cell culture obtained or obtainable by the inventive methods.

Also provided is a method of modifying expression of a gene of interest, such as by knockout or knockdown, in a haploid cell and observing modified activity of the cell related to said gene of interest.

In a further method of genetic analysis and mutagenesis, the inventive cells can be used in classical forward genetics. Random mutations, especially insertional mutations, can be introduced in the genome of the haploid cells, which result in a different phenotype, which in turn may be analysed in selected cells. The mutations may result in increase or decrease in gene expression. A decrease, including a complete ablation of expression, may occur when a gene is disrupted by the mutation. An increase is possible when an e.g. promoter activity is increased or a repressor becomes disrupted or removed from a gene of interest.

Usability of the inventive haploid cells can be increased by introduction of expression cassettes or expression reporters. Therefore the present invention provides a haploid mammalian cells, preferably as obtained as described above, comprising an expression cassettes or expression reporter. Further provided is a method of generating such cells, comprising obtaining a haploid mammalian cell, preferably as obtained as described above, and introducing an expression cassettes or expression reporter. Examples of expression reports include positive or negative selection markers, such as antibiotics resistance genes, HPRT1, diphtheria toxin, bioluminescent proteins. The expression reporters can be driven by a pre-selected promoter that activates at a certain condition of interest. Such a condition may be a stage of development of the cell, especially a degree of differentiation or being a certain cell type. Such promoters may be activated by the occurrence of certain DNA modifications of the genome, preselected DNA portions, in particular transgenes, such as BAC transgenes, or by integration of a certain genetic element of interest into a certain gene locus. Such a genetic element may be a knock-out construct as will be detailed below.

Thus the invention also relates to a method of generating cells comprising a phenotype of interest, comprising randomly mutating a plurality of cells with a haploid genome, preferably mammal cells, especially preferred cells obtainable or obtained by the inventive method, and selecting cells having the phenotype of interest.

Such a phenotype of interest is e.g. cell survival or cell growth, especially when said cells are contacted with a toxin or growth inhibitor. Thus cells with modified genes involved in resistance to said toxin or growth inhibitor can be generated. The genetic mutation can then be analysed and said gene identified.

Such a toxin is e.g. ricin. With the inventive methods it was possible to identify gene Gpr107 as a molecule essential for killing by ricin, a toxin being used as bioweapon, by such a forward genetic screen. Based on this proof-of-principle it is now of course also possible to generate resistant cell and identify relevant genes against any other toxin or growth inhibitor or other agents that induce a changed phenotype.

The inventive introduction of random mutations is preferably non-site specific and can potentially target any site in the genome. Preferred mutation inducers are retroviral or lentiviral infections or the used of nucleotide constructs capable of insertion in the genome, such as transposons. Of course minor site preferences of such insertion constructs or viral mutagens may exist, but in general most genes may potentially be targeted. Usually the inventive mutation is a gene disruption, preventing or reducing gene expression of a functional gene product. Further random mutations can be effected by contacting the cells with mutagenic chemicals or exposure to radiation.

For efficient random mutagenesis, a plurality of cells is used in these experiments, each cell potentially gaining a different mutation. Preferably at least 1000 cells, especially preferred at least 5000, at least 10000, at least 50000, at least 100000, at least 200000, at least 500000 or at least 1000000, cells are used.

Also provided is a cell obtained or obtainable by said method having a resistance to a toxin or growth inhibitor. E.g. there is provided a haploid cell having a resistance to a toxin of interest, preferably ricin.

The invention also provides a method of screening a cell for genetic targets having activity in toxin resistance comprising generating cells with a toxin or growth inhibitor resistance further comprising identifying a mutation in said cells having toxin resistance as compared to cells without said random mutation.

When a gene of interest, that needs modification, especially inhibition but also increased expression, has been identified by the inventive random screen it is also possible to modify other cells by modifying expression of the identified gene, e.g. by knock-out or knock-down mutations (for expression decreases) or introduction of expression vectors comprising said gene (for expression increase). Such genes have been identified for ricin resistance, which include genes selected from Gpr107, Fut9, Tcf711, Slc35c1, Fgfr2, Galnt2, Mid1, B4galt1, B4galnt3, Plcd3, Ror2, Samd4b, Gcnt2, Ggta1. These genes can be inhibited, e.g. by knock-out or knock-down, to generate cells with increased resistance to ricin. Such generated cells may be haploid cells, such as described above - that have been used to identify these targets, but may also be other cells, including diploid cells. Cells, including diploid cells may be from the same animal sources as described above for the inventive haploid cells.

Thus the present invention also provides a method of modifying a mammalian haploid cell, preferably as obtained by the inventive method described above, and inhibiting a gene of interest or modifying a genetic locus. Preferably such inhibition is by knock-out mutation, e.g. facilitated by homologous recombination of an inactivating gene construct that prevents expression of the gene of interest, or by modifying the gene of interest by using sequence specific nucleases, or by knock-down, especially using inhibitory nucleic acids for RNAi, including siRNA or shRNA. Genes of interest might or may not have been previously identified by an inventive random mutagenesis screen with a selection for a certain phenotype of interest. Genes of interest may be selected from Gpr107, Fut9, Tcf711, Slc35c1, Fgfr2, Galnt2, Mid1, B4galt1, B4galnt3, Plcd3, Ror2, Samd4b, Gcnt2, Ggta1 or any combination thereof. Also provided is a mammalian haploid cell obtainable by this method, comprising an inhibition of a gene as compared to a non-modified parental mammalian haploid cell.

In particular, the invention provides a modified cell with increased resistance to ricin comprising a reduced expression in any one of Gpr107, Fut9, Tcf711, Slc35c1, Fgfr2, Galnt2, Mid1, B4galt1, B4galnt3, Plcd3, Ror2, Samd4b, Gcnt2, Ggta1, or combinations thereof, as compared to an unmodified cell (e.g. parental cell of the same type without modification). Said modification can be by knock-out or knock-down, especially preferred by administration of an inhibitory nucleic acid, such as one that is or encodes an inhibitory RNS, including siRNA or shRNA. Such a modified call may be a haploid cell, especially a cell as obtained by the present invention or any other cell. Preferably the cell is a mammal cell and/or a non-human cell. In other embodiments the cell may be a human cell. Preferably the cell is an isolated cell and/or in vitro or ex vivo. In other embodiments, the cell may be in vivo, e.g. in a living organism. The cell may be haploid, e.g. as obtained by the inventive method, or diploid. Cells, including diploid cells, may be from the same animal sources as described above for the inventive haploid cells.

As used herein, "ex vivo" cell culture refers to culturing cells outside of the body. Ex vivo cell culture includes cell culture in vitro, e.g., in suspension, or in single-or multi-well plates. Ex vivo culture also includes co-culturing cells with two or more different cell types, and culturing in or on 2-or 3-dimensional supports or matrices, including methods for culturing cells alone or with other cell types to form artificial tissues.

The invention further relates to a method of identifying a therapeutic agent against a toxin, comprising identifying a genetic target as mentioned above and contacting a therapeutic candidate molecule with said genetic target or the gene product of said target, preferably in an isolated cell, and identifying binding events of the candidate with the genetic target or gene product or modified resistance of the cell to the toxin. The toxin is preferably ricin and preferably the genetic target is selected from Gpr107, Fut9, Tcf711, Slc35c1, Fgfr2, Galnt2, Mid1, B4galt1, B4galnt3, Plcd3, Ror2, Samd4b, Gcnt2 or Ggta1. But of course other toxins or growth inhibitors can be selected to identify genetic targets that are involved in resistance. In principle it is possible to test any kind of compound on its activity for being a therapeutic agent against a toxin by challenging a cell culture according to the invention. Once a genetic target (including its gene product, the encoded proteins) has been identified according to the invention, it is possible to directly test compounds for their activity to bind modulate, increase or decrease, especially inactivate these genetic targets, including the encoded proteins. Since genomic sequencing projects are complete for most organisms of interest, the sequence of the target gene is known once the genetic target has been identified. It is then easy to screen for candidate inhibitors that target these genes or the transcribed mRNA. Thus candidate nucleotide inhibitors can easily be screened. Inhibitors of proteins can be obtained by contacting a compound library to identify active compounds that bind the proteins. Toxin resistance activity of binders may then be confirmed by analysing the activity on the proteins or the cells or cell cultures of the invention directly. A preferred class of inhibitors targeting the proteins are antibodies. Antibodies against these target proteins can easily be generated by immunizing model animals, such as mice, rats, other rodents, like hamsters or rabbits, goats, etc. Antibodies as used herein also encompass antibody fragments that bind the target protein, including fragments with a Fv portion, including F(ab'), F(ab) or F(ab)₂ antibodies.

The invention also relates to a method of treating ricin poisoning in a subject or in a cell or inducing a ricin resistance in a subject or in a cell comprising inhibiting any one of Gpr107, Fut9, Tcf711, Slc35c1, Fgfr2, Galnt2, Mid1, B4galt1, B4galnt3, Plcd3, Ror2, Samd4b, Gcnt2 or Ggta1 to said subject.

Inhibiting can be by administration of an inhibitor. An inhibitor of said genes relates to a compound that decreases the level of expression or activity of said genes, or their respective gene products, including the expressed proteins. Said inhibitor can be an antibody or an inhibitory nucleic acid, especially comprising or encoding a small inhibitory RNA molecule, especially preferred an inhibitory nucleic acid that reduces mRNA expression or content of said genes by at least 20%, especially by at least 50%, in a cell. Nucleotide inhibitors can be selected from antisense molecules, ribozymes or small inhibitory RNA molecules (small interfering RNA (siRNA); in the meaning of the present invention, regulatory RNAs such as "micro RNA" ("miRNA") and "short hairpin RNA" ("shRNA") are used interchangeably with the term "siRNA"). Inhibition can be by reducing expression, protein content or activity.

A specific embodiment of nucleotide-type inhibitors employs the application of RNA interference (RNAi). RNAi is the process of sequence-specific post-transcriptional gene silencing initiated by double-stranded RNA that is homologous in sequence to the silenced gene. Small interfering RNA (siRNA) duplexes of 21 to 22 nucleotides are shown to be a powerful tool for inhibiting gene function in mammalian cells. Vector-based systems for stable expression of short interfering RNAs are preferred. These systems are based on a vector, in which a synthetic, gene-specific target sequence encoding the siRNA is expressed under the control of a promoter that is suitable for transcription of small, non-coding RNA. The siRNAs are thus produced from the vector following its introduction into mammalian cells by standard transfection (e.g. electroporation, lipofection) or viral infection protocols (e.g. retroviral infection). A suitable small interfering RNA molecule can be easily selected by known methods in the art. E.g. common is the synthesis of a plurality of about 20-50 different said duplexes of 21 or 22 nucleotides in length and contacting test cells. Cells with the most reduced expression of the target gene are selected. According to the present invention, it is of course also possible to use the phenotype of the haploid cells, e.g. toxin or growth inhibitor resistance, as a marker to select the best inhibitory RNA molecules. Methods for selecting designing siRNAs, including selection of the targeted sequence, preparation of the siRNA duplexes, vector design and delivery are well known in the art, e.g. described in detail in US 7,235,654.

As an alternative to siRNA, antisense oligonucleotides can be used as nucleotide-type inhibitors to interfere with the expression of the target genes, gene products or proteins. Antisense oligonucleotides are short stretches of nucleotides that are complementary to a region of the target mRNA and can specifically suppress expression of that particular transcript. The antisense nucleic acid can take the form of RNA expressed from a vector, which has been transfected into the cell or take the form of a DNA or RNA oligonucleotide which can be introduced into cells through a variety of means, e.g. by means of cationic liposomes, cationic porphyrins, fusogenic peptides, and artificial virosomes, or cell permeabilization with streptolysin-O and electroporation. Cationic lipids form stable complexes with oligonucleotides, which exhibit improved cellular uptake, thus resulting in enhanced antisense activity.

In a further embodiment, the invention relates to ribozymes directed against a target gene, gene product or protein. Similarly to antisense oligonucleotides, ribozymes bind to substrate RNA through Watson Crick base pairing, which leads to sequence-specific cleavage of transcripts. Two types of ribozymes, the hammerhead ribozyme and the hairpin ribozyme, have been extensively studied due to their small size and rapid kinetics. Their application has been reviewed in several publications. Ribozymes can be imported into the cell by various means, as described above for antisense oligonucleotides, or they can be expressed from a vector, which offers the advantage of continued intracellular production of these molecules.

Preferably, the nucleotide-type inhibitors are produced from a viral vector, e.g. a retroviral or adenoviral vector, an adeno-associated viral vector or lentiviral vector.

In one embodiment, nucleotide-type inhibitors or constructs encoding the nucleotide-type inhibitors are delivered to cells by transfection, i.e. by delivery of "naked" DNA or in a complex with a colloidal dispersion system. A colloidal system includes macromolecule complexes, nanocapsules, microspheres, beads, and lipid-based systems including oil-in-water emulsions, micelles, mixed micelles, dendrimers and liposomes. A colloidal system may be a lipid-complexed or liposome-formulated nucleotide-type inhibitor. Formulation of the inhibitor, e.g. with various lipid or liposome materials, may then be effected using known methods and materials and delivered to the recipient cell or mammal.

Such inhibitors may be administered in form of a pharmaceutical composition. Pharmaceutical compositions may comprise further carrier, including buffers, emulsifiers, and/or additives suitable for a specific mode of administration. The pharmaceutical compositions can be administered systemically or locally, such as, but not limited to, by direct injection into the tissue of interest. The compositions are in general administered intravenously, intramuscularly, as implants, or topically. Suitable pharmaceutical preparation forms are, for example, injectable solutions in ampule form, emulsions, suspensions, creams, aerosols, preparations with sustained release of active compounds.

The present invention is further illustrated by the following figures and examples, without being restricted to these embodiments of the invention.

### Figures:

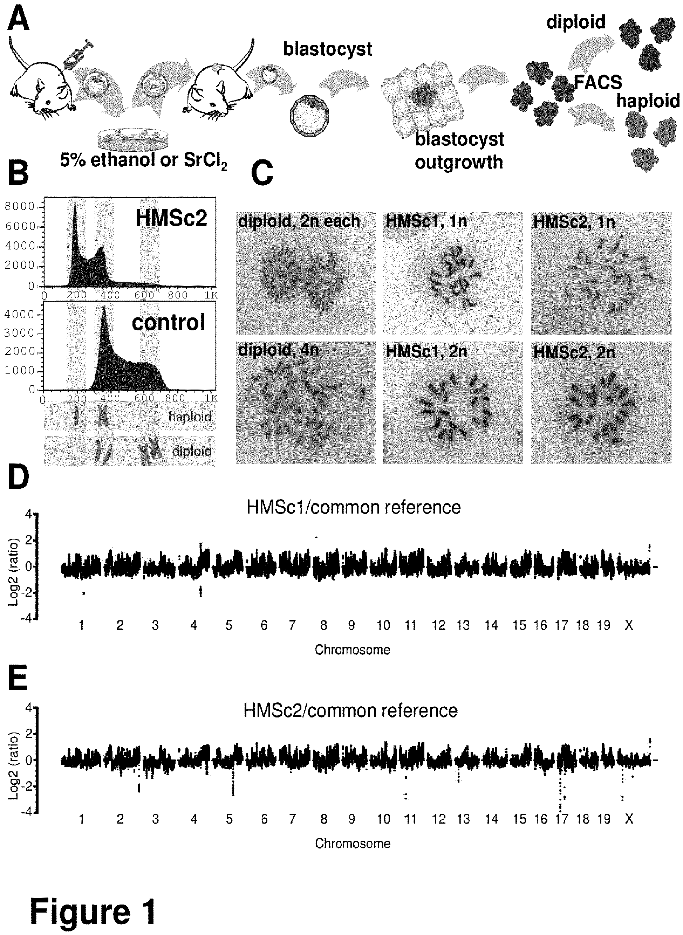

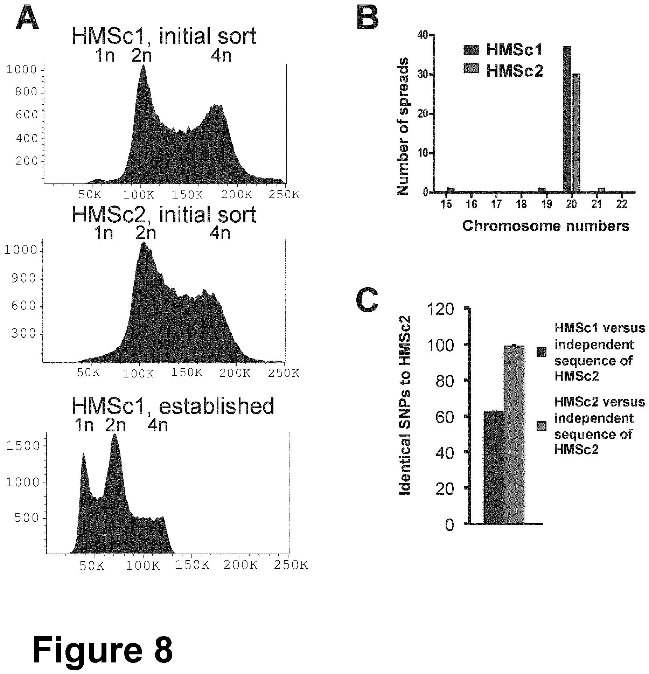

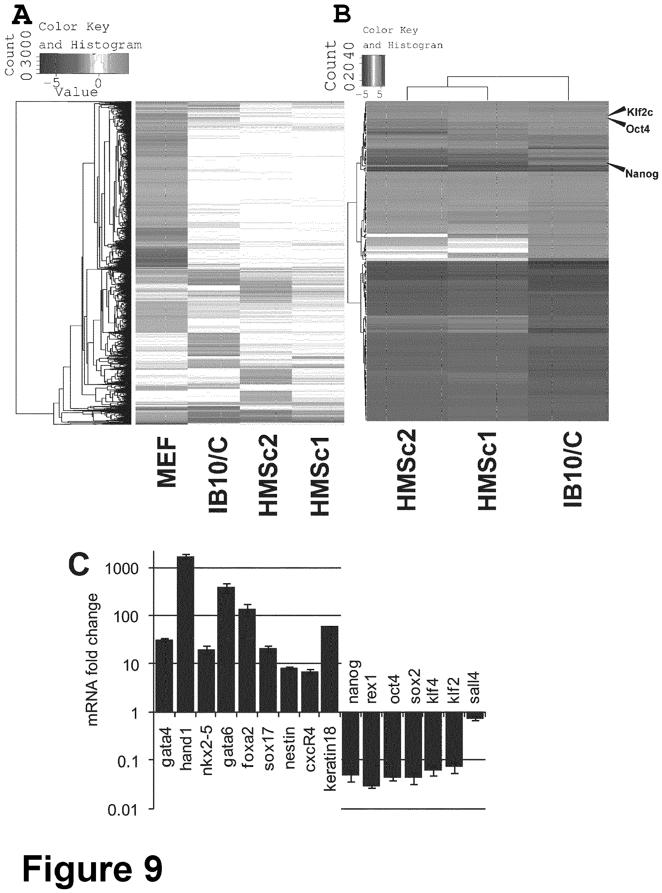

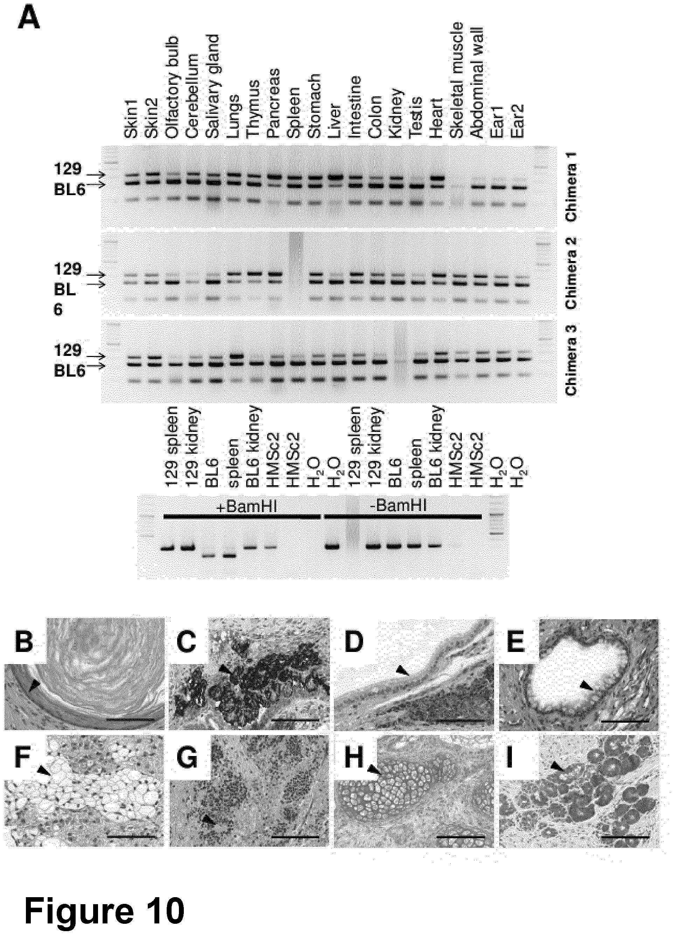

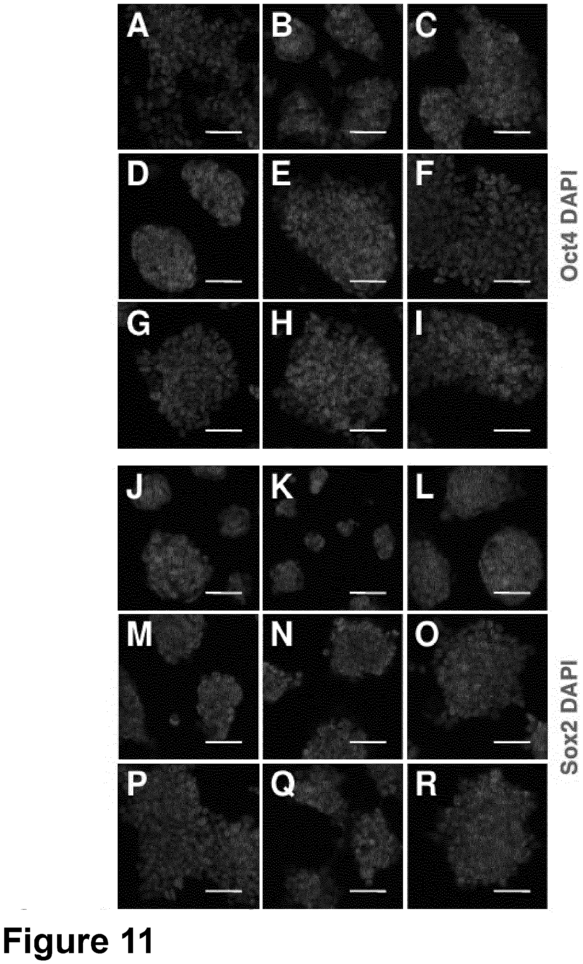

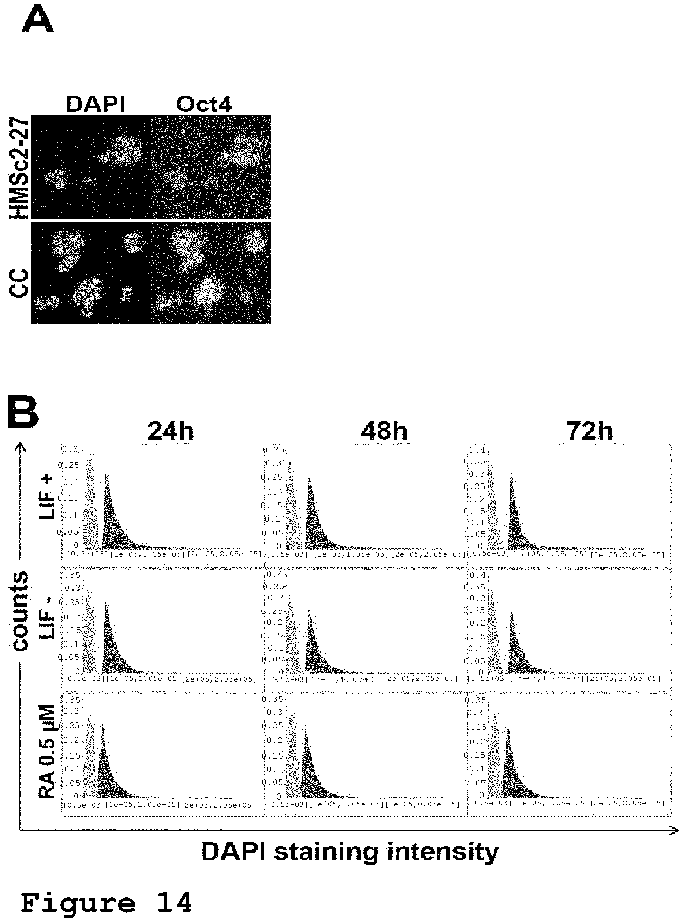

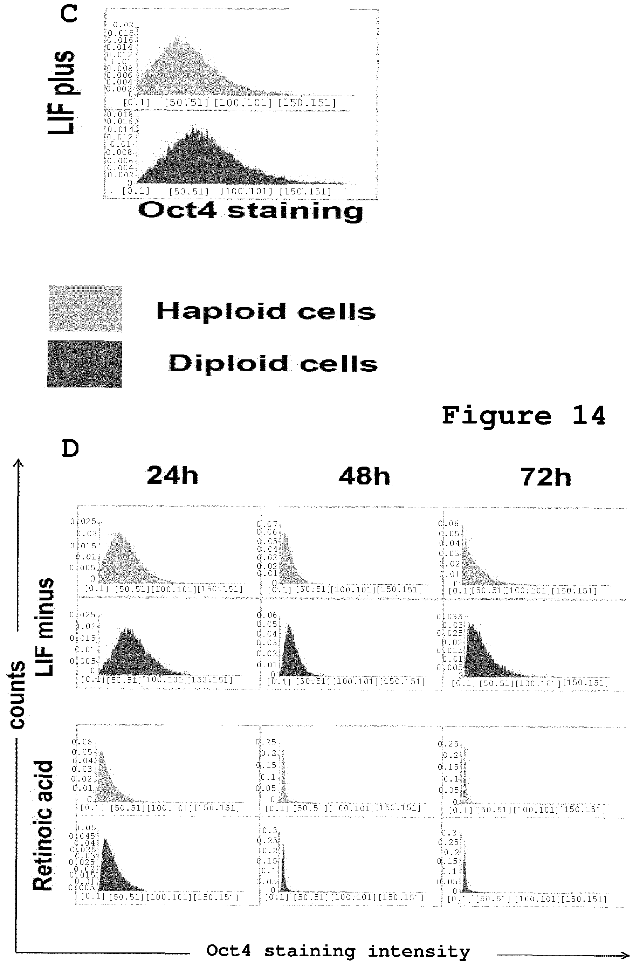

**Figure 1****. Generation of haploid murine stem cell lines.**
   **A)** Schematic overview of induction of parthenogenesis and the derivation of haploid stem cell lines. Mouse oocytes were activated with either 5% ethanol (or 25 mM Strontium Chloride (SrCl₂)) and implanted into pseudopregnant females. Stem cells were then generated from blastocysts and haploid cells subsequently sorted by FACS. Cultures were routinely resorted until stable haploid cells were derived. **B)** Flow cytometric analysis of DNA content in the control diploid stem cell line IB10/C and the haploid HMSc2 cell line. DNA content was determined using Hoechst33342. 1n and 2n chromosome sets for haploid and 2n and 4n chromosome sets for diploid stem cells are indicated. The histograms show data from cells at the 10^{th} sort. **C)** Representative chromosome spreads of control diploid stem cells and haploid HMSc1 and HMSc2 cells. Spreads from anaphase (1n) and prophase (2n) of mitosis are shown for haploid cells. As a control, anaphase (2n) and prophase (4n) spreads are shown for diploid stem cells. **D, E)** Sequence coverage relative to the common reference of parental in-house C57BL/6 and 129 strains is shown on a log2 scale. Haploid cells were derived from C57BL/6x129 crosses. Chromosomes are arranged in numerical order and separated by small gaps. See also Figure 8.

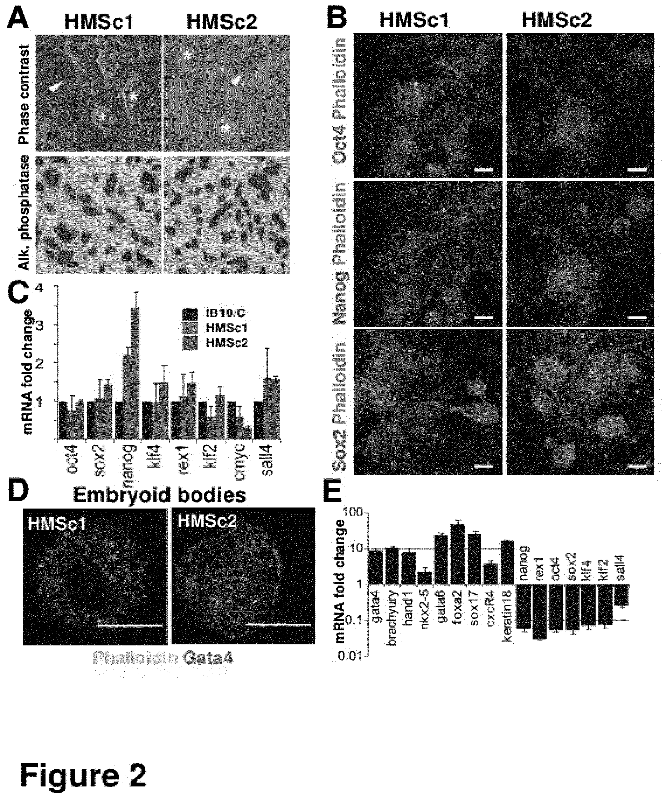

**Figure 2****. Marker analysis and *in vitro* differentiation potential of haploid stem cell lines.**
   **A)** Both haploid HMSc1 and HMSc2 cell lines exhibit a morphology characteristic of stem cell colonies (asterisk). Representative phase contrast images are shown. Note the feeder layer of mouse embryonic fibroblasts (MEF) (arrowheads). Haploid cells stain also positive for the stem cell marker alkaline phosphatase (blue, bottom panels). **B)** Expression of Oct4, Nanog, and Sox2, prototypical markers for murine embryonic stem cells. Phalloidin staining indicates the feeder cell layer. Haploid HMSc1 and HMSc2 cell were co-stained for Oct4 (FITC) and Nanog (TRITC). In both cases, stainings are shown separately in the red channel. Scale bars are 50µm. Data are from cells after the 4^{th} sort. **C)** Expression of prototypic stem cell marker genes in the haploid HMSc1 (blue) and HMSc2 (red) cells. mRNA expression was determined using qPCR and normalized to diploid IB10/C ES cells (black bars). **D)** Gata4 protein expression in embryoid bodies (EB, day 7) as a marker for endoderm. Representative EBs are shown for both haploid HMSc1 and HMSc2 ES cell lines counter-stained with phalloidin (green). Scale bars are 50µm. **E)** qPCR revealed down-regulation of the stem cell markers Nanog, Rex1, Oct4, Sox2, Klf2, Klf4, and Sall4 in EBs (day 7) derived from the haploid stem cell line HMSc2 accompanied by expression of the indicated lineage commitment markers (see text). mRNA expression was normalized to the parental, undifferentiated haploid ES cells (set at 1). See also Figure 9.

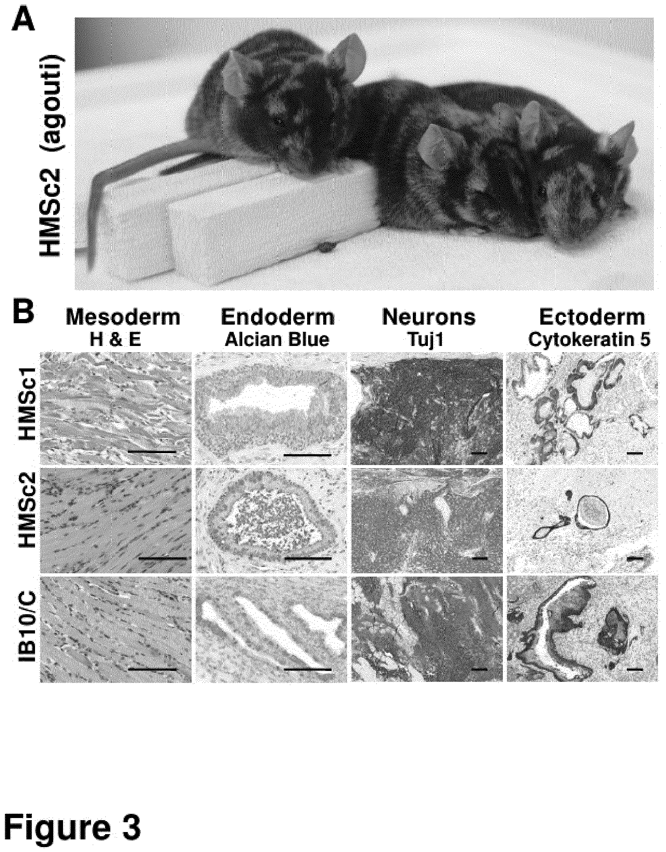

**Figure 3****. *In vivo* differentiation potential of haploid stem cell lines.**
   **A)** Haploid stem cells can contribute to tissues in adult mice. Diploid cells from the Agouti⁺ clone HMSc2 were injected in into C57BL/6 blastocysts and coat color chimerism was observed (brown fur patches). **B)** Histological and immunohistological analysis of teratomas derived from control diploid IB10/C stem cells and the haploid ES cell lines HMSc1 and HMSc2. Haploid stem cells can contribute to all three germlayers, namely muscle cells (H&E), intestinal endoderm (mucin producing goblet cells stained by Alcian blue, counterstained with nuclear fast red), Tuj1⁺ neurons, and Cytokeratin 5 (K5) expressing ectoderm. Tuj1⁺ and K5⁺ cells were detected by immunohistochemistry (DAB, brown), counter-stained with hematoxylin (blue). Scale bars: 100µm. See also Figure 10.

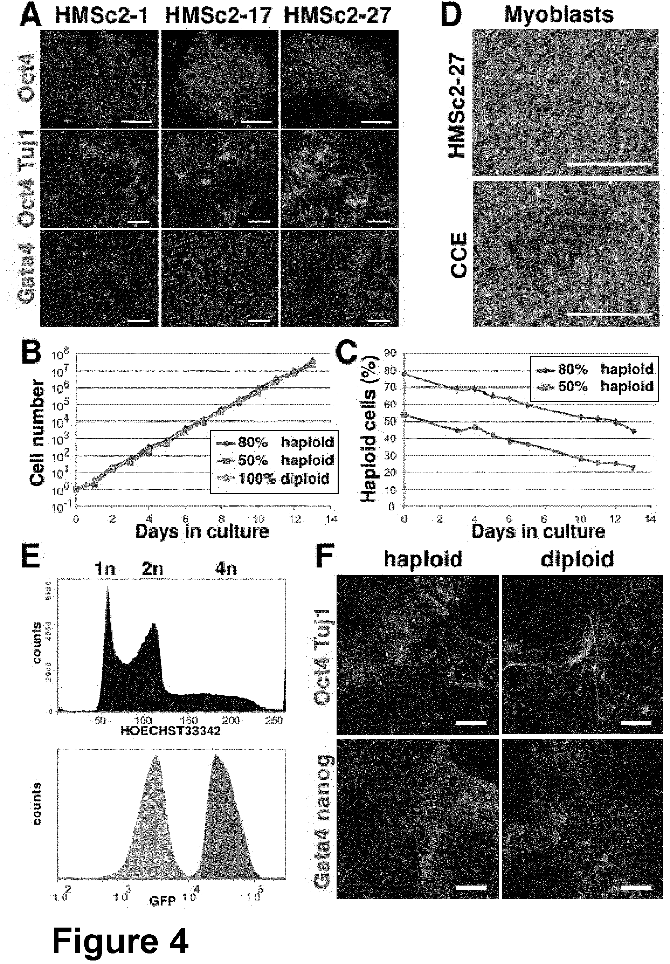

**Figure 4****. Haploid stem cells have the intrinsic ability for stable growth and differentiation.**
   **A)** Immunostaining for Oct4 protein expression (red) on three different subclones that were established by plating single haploid cells directly after FACS purification (top panels). The middle and bottom panels show immunostaining for Oct4 (red) and Tuj1 (green) expression and expression of the endodermal marker Gata4 (red, counterstained with DAPI) in attached embryoid bodies (EBs, day 10) derived from the indicated subclones. Data are from cells that were subcloned after > 30 passages of the parental line. Scale bars are 50µm. **B)** Proliferation rates and **C)** percentages of haploid cells in control cultures containing 100% diploid MHSc2-27 cells and cultures of HMSc2-27 cells seeded at 80:20 and 50:50 ratios of haploid:diploid cells. Multiplication rates and percent haploidy were determined every 24 hours using FACS analysis of Hoechst33342 stained cells. Note that for this experiment cells were continuously kept in culture for 7 passages (14 days). Based on this experiment, we estimate that ∼2-3% of haploid cells became diploid each day over the course of the experiment. **D)** Development of myoblasts from the haploid ES cell subclone HMSc2-27. The feeder cell free diploid stem cell line CCE was used as a control for this experiment. Representative phase contrast images are shown. Scale bars are 100µm. **E)** GFP expression (green) in a GFP-tagged haploid stem cell subclone. Non-GFP labeled cells are shown as control (grey shaded histogram). Flow cytometry of DNA content (Hoechst33342) is shown for the same subclone demonstrating that both haploid and diploid cells express GFP. **F)** Differentiation of haploid and diploid HMSc2-27 cells into EBs (day 13) that contain Tuj1 neurons (green) and Gata4 expressing endodermal cells (red). Note downregulation of Oct4 expression (red, upper panel) and the presence of residual clusters of Nanog⁺ cells (green, bottom panels). In the top panels, cells were counterstained with DAPI to visualize nuclei. See also Figure 11-13.

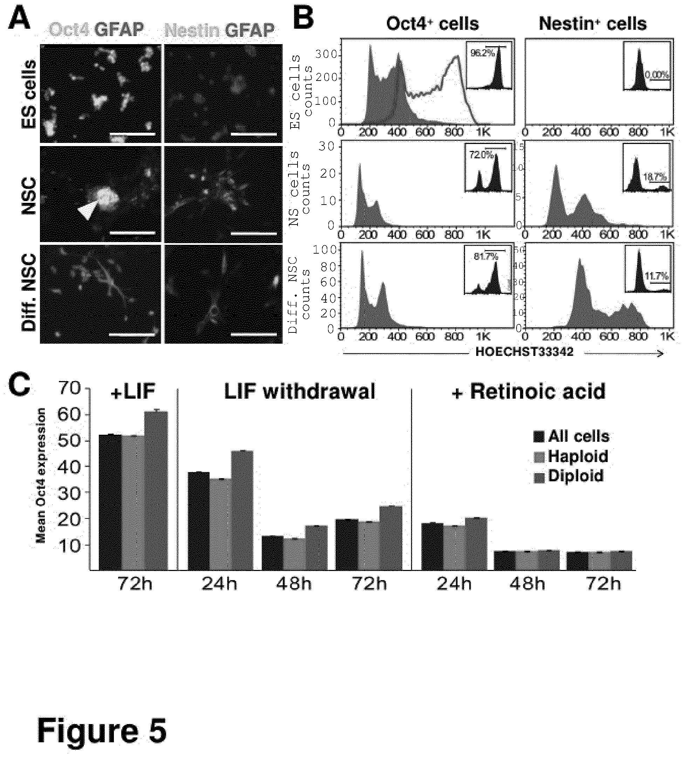

**Figure 5****. Differentiation potential of haploid stem cells.**
   **A)** Analysis of the haploid stem cell clone HMSc2-27 cultured under conditions to maintain an stem cell fate ("stem cells"), *in vitro* differentiated into Nestin⁺ neural stem cells (NS cells), and further differentiation into GFAP⁺ astrocytes by withdrawal of EGF and FGF2 in the presence of 1% serum (differentiated NS cell culture conditions). Immunofluorescence labeling of Oct4, Nestin, and GFAP are shown, counterstained for DAPI. Representative imagines are shown. Scale bar is 100µm. **B)** Flow cytometry analysis of DNA content in cells gated for Oct4 and Nestin expression and grown under stem cell (top), NS cell (middle), and differentiated NS cell (bottom panels) conditions. The gates used and percentages of cells are inserted in each plot. Haploid cells are prominent in Oct4⁺ fractions under all conditions while Nestin⁺ cells differentiated for 4 days are devoid of haploid cells. The red line in the top left panel shows the representative DNA content of the diploid control IB10/C stem cell line gated for Oct4 expression. **C)** Haploid cells exit the pluripotent state following the same dynamics as diploid cells. The left panel shows control Oct4 levels (mean intensity depicted) in haploid HMSc2-27, mixed (haploid and diploid) HMSc2-27, and control diploid CCE stem cells after 72h under control (plus LIF) conditions. Differentiation by LIF withdrawal leads to diminished Oct4 expression of diploid and haploid cells (middle panel). Differentiation induction using 0.5µM retinoic acid results in a rapid loss of Oct4 expression in both haploid and diploid cells, indicative of differentiation (right panel). The same results were obtained when we used 0.1µM retinoic acid (not shown). Data are shown as mean Oct4 fluorescence intensity +/-SEM analysing more than 50.000 cells per condition. 1way ANOVA (p>0.05) showed increased expression of Oct4 in diploid cells (consistent with increased nuclear area) in all conditions except the 48- and 72-hour retinoic acid treatments wherein Oct4 expression was at background levels. See also Figure 14.

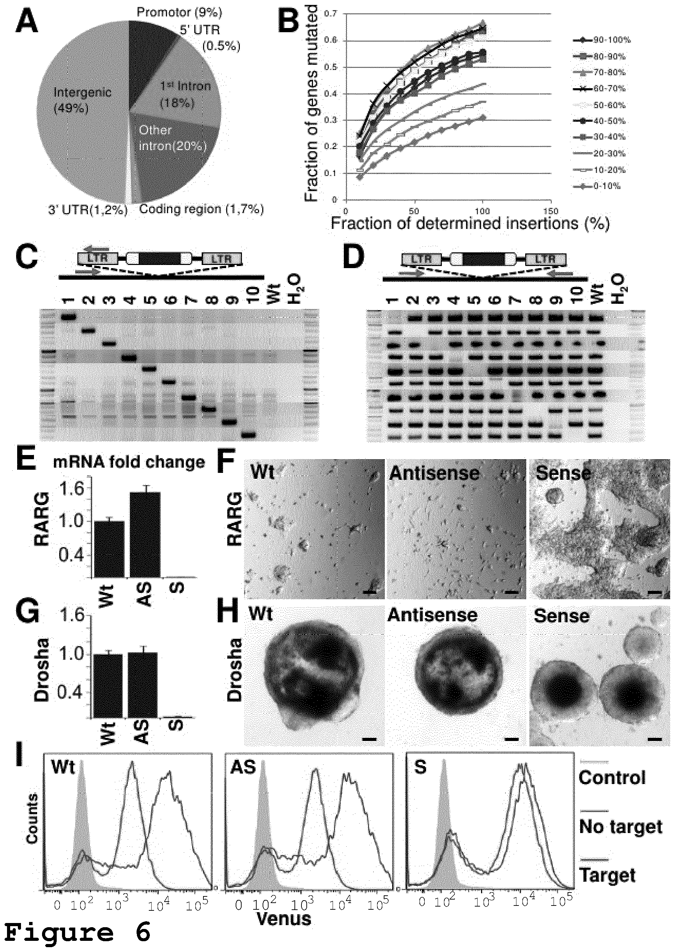

**Figure 6****. Reverse genetics in haploid stem cells.**
   **A)** Analysis of virus integration sites after neomycin selection. 176,178 insertions were determined by deep sequencing. The retrovirus landed in 49% in intergenic and in 51% in intragenic regions, with a high frequency of integration into introns, especially the first intron. **B)** Graph shows percentage of genes with virus integrations following a single round of retroviral mutagenesis for different fractions of the total viral integration sites (X-axis). Genes with the 10% lowest expression (0-10%) showed the least integration efficiency while higher expressed genes (50-100%) show more efficient gene trapping. For increasing fractions of the total viral integration sites (X-axis) higher saturation is reached, up to 67% yet not reaching saturation, indicating that additional genes are trapped in the total library of 7.5 million independent insertions. **C,D)** PCR analysis using site specific primers for the indicated genes and a primer specific for the LTR of the inserted retrovirus. The location of the used PCR primers is schematically indicated on top of each panel. Of note, all primers were used for all 10 different genes showing **(C)** that the virus has indeed integrated into the site identified by initial sequencing and **(D)** that the integrations result in homozygous mutations of the respective loci. Lane 1 = *Madcam1*; lane 2 = *Drosha*; lane 3 = *Retinoic acid receptor gamma* (*Rarg*); lane 4 = *Ap4s1*; lane 5 = *Arap1*; lane 6 = *Evx1*; lane 7 = *Bcl2l1*; lane 8 = *2210012G02RIK*; lane 9 = *Titin*; lane 10 = *Chr2:50928851*. Positive wild type (Wt) and negative H2O controls are shown. **E)** qPCR analysis of RARG mRNA expression in haploid HMSc2-27 cells that are wild type for *rarg* (Wt), HMSc2-27 cells that contain the splice acceptor in antisense orientation (AS), and HMSc2-27 cells that contain the splice acceptor in the sense orientation (S). mRNA expression was normalized to the parental HMSc2-27 cells. **F)** Representative images of cultures containing the indicated wild type, antisense, and sense RARG HMSc2-27 cells treated with 0.1µM retinoic acid for 10 days. Note the near complete absence of cells in the Wt and antisense cultures. Scale bars are 100µm. **G)** qPCR analysis of Drosha mRNA expression in haploid HMSc2-27 cells that are wild type for *Drosha* (Wt), HMSc2-27 cells that contain the splice acceptor in antisense orientation (AS), and HMSc2-27 cells that contain the splice acceptor in the sense orientation (S). mRNA expression was normalized to parental HMSc2-27 cells. **H)** Complete absence of cystic embryoid bodies in *Drosha* deficient HMSc2-27 cells as compared to Drosha expressing wild type HMSc2-27 cells and cells containing the splice acceptor in the antisense orientation. Representative images for embryoid bodies are shown on day 10 after EB induction. Of note, a single cystic EB in *Drosha* mutant cells even in prolonged culture was not observed. Scale bars are 100µm. **I)** Histograms showing Venus reporter gene expression in wild type HMSc2-27 cells (Wt), HMSc2-27 cells that contain the splice acceptor in antisense orientation (AS), and HMSc2-27 cells that contain the splice acceptor in the sense orientation (S) transduced with pSENSOR-based miRNA constructs harboring a potent shRNA targeting Firefly Luciferase with (target) or without its target (no target) site in the 3'UTR of Venus. Cells were gated on shRNA expressing (dsRed⁺) cells and Venus expression levels were compared to non-transduced control cells (grey).

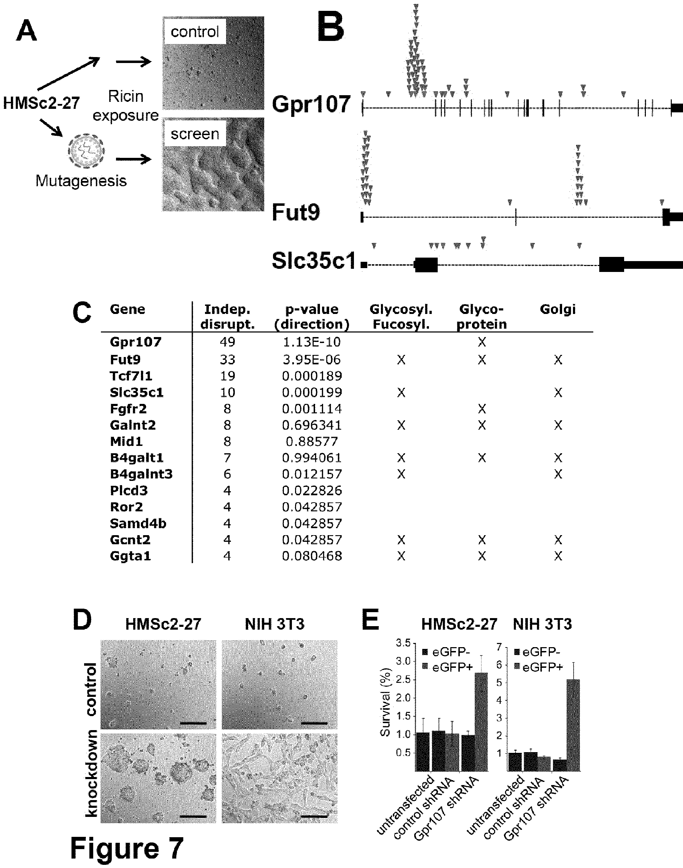

**Figure 7****. Forward genetic screen for ricin toxicity in haploid stem cells**
   **A)** Haploid HMSc2-27 with/without gene-trap mutagenesis was exposed to ricin from *Ricinus communis* for 3 weeks. Colonies only appeared in the mutagenized batch and were processed for deep sequencing. **B)** Top hits identified in the ricin toxicity screen. Sense (green) and antisense (red) insertions in *Gpr107*, *Fut9*, and *Slc35c1* genomic loci. The vertical lines indicate the respective exons for each gene with the first exon always moved to the left side of each diagram. Insertions in antisense might disrupt gene function, sense integrations will do so in almost all cases. Note that nearly all insertions are in sense for the splice acceptor and that some antisense integrations map to exons, all of which should result in disruptive mutations. Considering that ∼ 50% of intragenic insertions are sense and ∼ 50% in antisense, these data also show that the screen has indeed strongly enriched for disruptive mutations (p>1.13E⁻¹⁰ for *Gpr107*; p>3.95E⁻⁶ for *Fut9*; p>0.000019 for *Slc35c1*). **C)** Genes identified in the ricin toxicity screen. The numbers of distinct retroviral insertions predicted to disrupt gene expression (either because intragenic regions containing the sense orientation of the splice acceptor, or sense and antisense integrations into exons) are indicated. Enrichment for sense mutations vs. anti-sense integrations was assessed using a binomial test and the respective p values are indicated. Of note, anti-sense integrations can also lead to gene disruption. Assigned biochemical pathways and allocation to the Golgi apparatus are also indicated. **D, E)** Validation of Gpr107 in Ricin toxicity. HMSc2-27 stem cells and NIH3T3 cells were transfected with shRNAs designed to knockdown Gpr107 expression or transfected with control shRNA and then challenged with a lethal dose of ricin for 2 days. Images show representative cultures after 48 hours of ricin treatment **(D)**. Scale bars are 100µm. **(E)** The ricin survival rate as ratio between recovered cells of ricin treated vs. ricin untreated cells is shown in % (as determined by quantitative FACS analysis of cells gated for viability by forward scatter, side scatter, and PI staining after 48 hours of ricin treatment). Cells were cultured in 10cm dishes in triplicates and average survival +/- SD was determined for eGFP negative (untransfected) and eGFP positive haploid HMSc2-27 ES cells and NIH 3T3 cells for each plate.
**Figure 8****. DNA content analyses during derivation of haploid**
   **cells, chromosome spread, and SNP analyses; refers to** **Figure 1****. A)** The first FACS analysis upon derivation of our two cell lines showed a small subpopulation of haploid cells in G1 of cell cycle (1n) derived from parthenogenotes. The initial sorts are shown. Upon repeated FACS purification (sort 7 is shown), a population of HMSc1 was enriched for haploidy (bottom panel). Of note, since HMSc1 cells always exhibited a larger number of diploid cells we primarily focused on HMSc2 cells. **B)** The vast majority of chromosome spreads displayed a precisely haploid genome (20 chromosomes). Rare spreads with changed chromosome number can be due to overlapping or washed off chromosomes. **C)** SNP comparison between HMSc1 or HMSc2 to an independent sequencing run of HMSc2 based on discriminatory SNPs between the 129 and C57BL/6 mouse strains. Haploid cell lines were derived from C57BL/6x129 F1 intercrosses. Data are shown as identical SNPs to HMSc2 confirming genetic independence of the two haploid clones (Student's t-test: p<5.788E-08).
**Figure 9****. Transcriptome analysis and lineage markers in embryoid bodies; refers to** **Figure 2****.**
   **A)** Haploid HMSc1 and HMSc2 display a gene expression profile that clusters with that of an established control ES cell line (diploid IB10/C ES cells). The transcriptome profile of MEFs is shown as control. Values are relative to the reference pool of all 4 RNA samples. **B)** Clustering shows that the transcriptional profile of both haploid HMSc1 and HMSc2 cells closely resembles that of diploid IB10/C ES cells. For clustering, the 100 most up- or down-regulated genes between MEFs and diploid IB10/C stem cells were selected. Three prototypical ES cell genes, namely Nanog, Oct4, and Klf2 are indicated. Supplementary Table 2 lists the 100 genes included in the analysis. For both A) and B), upregulated genes are shown in blue, downregulated genes are shown in red (see color keys), and hierarchical clustering of genes is shown as tree on the side of the heat maps. **C)** qPCR analysis reveals down-regulation of the stem cell markers Nanog, Rex1, Oct4, Sox2, Klf2, Klf4, and Sall4 in EBs (analysed on day 7) derived from the haploid stem cell line HMSc1 accompanied by expression of the indicated lineage commitment markers (see text). mRNA expression was normalized to undifferentiated haploid stem cells (set at 1).
**Figure 10****. Haploid stem cells can contribute to various tissues in adult mice and teratomas; refers to** **Figure 3****.**
   **A)** Contribution of the HMSc2 derived cells to multiple tissues was determined by PCR followed by BamHI digest using primers GAATGTGAGCGCACAGGGTGATGTGCC (SEQ ID NO: 1) and CCCACAGAACA-CAGTCACAGGGTCC (SEQ ID NO: 2). The indicated tissues were harvested from mice displaying coat color chimerism, processed, and analysed for the presence C57BL6 (BL6) and 129 specific bands. The presence of a 129 band indicates tissue contribution by HMSc2 cells. The specificity of the primers in the BamHI digests are shown in the bottom panel. **B-I)** Histological examination of teratomas stained with hematoxylin & eosin **(B-G)** or Alcian blue, counterstained with alizarin red **(H,I)**. Arrowheads point towards **B)** keratinized stratified epithelium, **C)** pigmented epithelium, **D)** ciliated respiratory epithelium, **E)** glandular tubules with goblet cells, **F)** adipocytes, **G)** neurotubules, **H)** cartilage tissue, and **I)** sweat gland structures. Scale bars are 100µm.
**Figure 11****. Oct4 and Sox2 expression on haploid stem cell subclones; refers to** **Figure 4****.**
   Immunostaining for Oct4 protein expression (red, top panels) and Sox2 protein expression (red, bottom panels) on 9 different subclones that were established by plating single haploid cells directly after FACS purification. Subclones were derived from both HMSc1 and HMSc2 haploid stem cells. The subclones HMSc1-N1 (A, J), HMSc1-N3 (B, K), HMSc2-N3 (C, L), HMSc2-N4 (D, M), HMSc2-N6 (E, N), HMSc2-1 (F, O), HMSc2-15 (G, P), HMSc2-17 (H, Q) and HMSc2-27 (I, R) were seeded on gelatin coated coverslips and immunostained for Oct4 and Sox2 expression. Data are from cells that were subcloned after > 30 passages of the parental line confirming stability of expression of bona fide stem cell markers. Cells were counter-stained with DAPI (blue). Scale bars are 50µm.

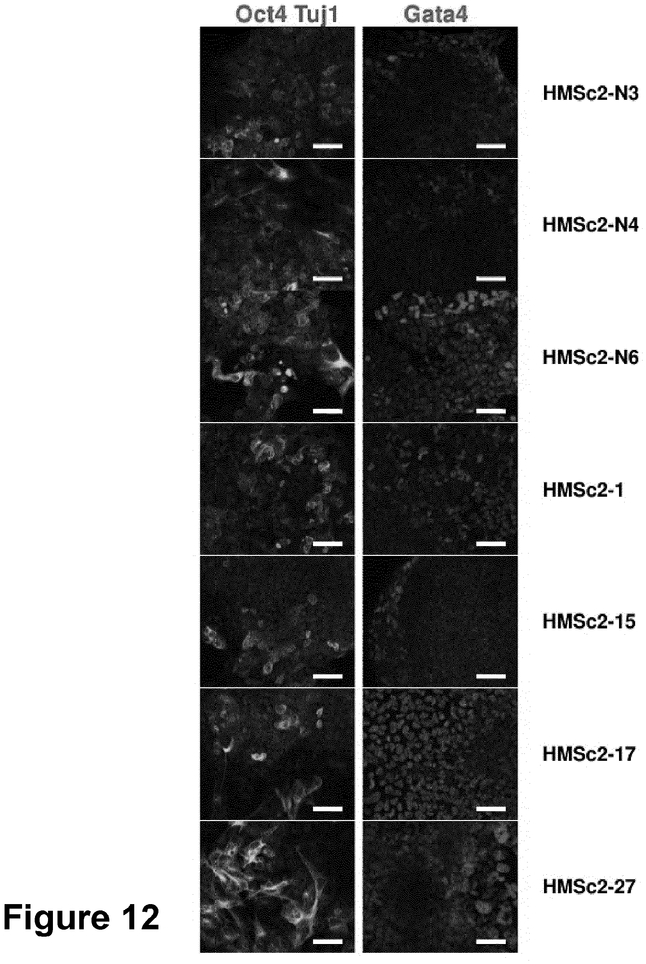

**Figure 12****. Developmental potential of haploid stem cell subclones; refers to** **Figure 4****.**
   Immunostaining for Oct4 (red) and Tuj1 (green) expression and expression of the endodermal marker Gata4 (red) in attached embryoid bodies (EBs, day 10) derived from the indicated subclones. Cells were counterstained with DAPI (blue). Data are from cells that were subcloned after > 30 passages of the parental line. Scale bars are 50µm.

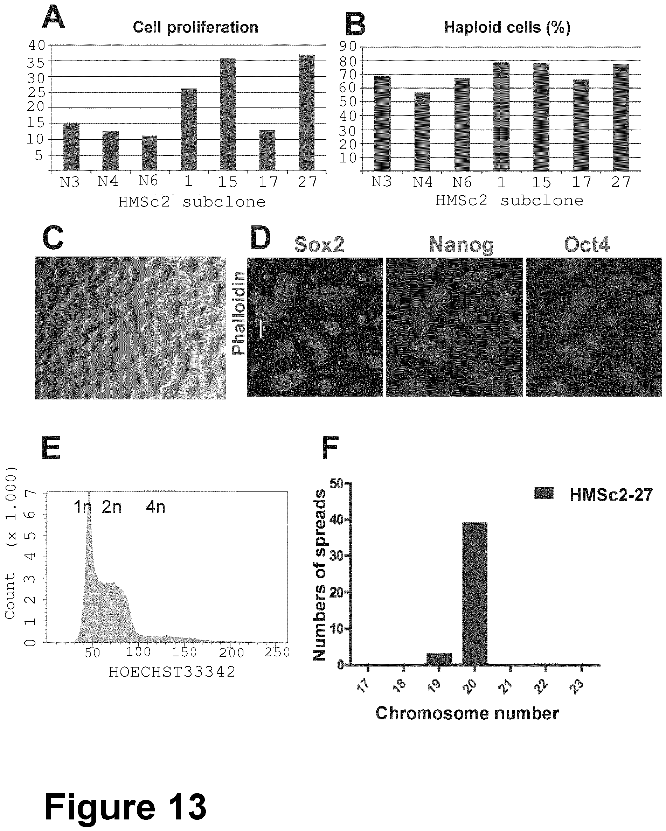

**Figure 13****. Proliferation and stability of the haploid cell fraction in different stem cell subclones; refers to** **Figure 4****.**
   **A)** Proliferative kinetics was monitored in 7 subclones derived from haploid HMSc2. 1x10⁶ cells were plated on a 10cm dish and cell numbers were determined 72 hours later. All clones displayed robust growth. Clones HMSc2-15 and HMSc2-27 reached >30 million cells within a 72 hour timeframe while the HMSc2-N4 and HMSc2-N6 completed more than 1 cell cycle less. **B)** Percentages of haploid cells present in the populations of seven different subclones derived from the haploid HMSc2 stem cell line. Upon FACS purification of a purely haploid population for all subclones (In peak as defined by Hoechst33342 histogram), relative populations of haploid versus diploid cells were determined after ten days of culture by FACS analysis on DNA content and quantified using the ModFit software. **C)** Morphology of the HMSc2 subclone HMSc2-27 cultured under feeder cell free conditions. HMSc2-27 cells remain a large haploid fraction for several weeks even without FACS sorting and exhibit the typical morphology of stem cells forming rounded, compact colonies. Representative DIC (differential interference contrast, Nomarski) images are shown. **D)** Expression of the prototypical murine embryonic stem cell markers Oct4, Nanog, and Sox2. Note absence of Phalloidin-positive feeder cells. Nanog and Oct4 were co-stained and are shown separately in the red channel. Scale bar: 50µm. **E)** Flow cytometry of DNA content in HMSc2-27 cells. DNA content was determined using Hoechst33342. The 1n and 2n chromosomes for haploid and 2n and 4n chromosomes for diploid stem cells are indicated. **F)** Chromosome spreads of HMSc2-27 cell confirming a haploid genome.
**Figure 14****. Separation of haploid and diploid cells using high content imaging analysis and quantitative assessment of Oct4 levels; refers to** **Figure 5****.**
   **A)** Representative high content scanning images of haploid HMSc2-27 and diploid CCE cells from 24 hour LIF withdrawal cultures. Parallel cultures of haploid HMSc2-27 and diploid CCE cells were used to develop an algorithm to distinguish haploid versus diploid cells on nuclear area (nuclear mask) and DNA content (DAPI staining intensity). The nuclear masks defined by DAPI staining are depicted in blue. Orange masks were rejected as nuclei from the algorithm as they are too small. The green lines in the Oct4 stained section cells depict nuclear masks as defined by DAPI staining. Of note, as expected, staining is less intense in the haploid cells for both DAPI and Oct4. **B)** Relative frequency plots showing separation of haploid (yellow) and diploid (blue) HMSc2-27 cells as a function of total DAPI intensity (x-axis) in the presence of LIF (top panels), the absence of LIF (bottom panels), and following treatment with 0.5µM retinoic acid (bottom) at 24h, 48h and 72h. To avoid inclusion of diploid cells in the haploid group and vice versa, only cells lying below the means of haploid controls were considered haploid, and only cells lying above the means of diploid controls are considered diploid by our algorithm. **C)** Baseline Oct4 expression in haploid versus diploid HMSc2-27 ES cells cultured in the presence of LIF. **D)** Oct4 expression of haploid versus diploid HMSc2-27 stem cells cultured in absence of LIF or in the presence of 0.5 µM retinoic acid to induce differentiation. Representative frequency histograms of Oct4 intensity corresponding to the data in Fig. 5C are shown. Note that at the time-points analysed haploid cells (yellow) display nearly identical differentiation dynamics to control diploid cells (blue). Oct4 intensity decreases over time under both differentiation conditions but particularly rapidly under retinoic acid treatment. Note that Oct4 expression decreases in haploid as well as diploid HMSc2-27 cells at comparable rates upon initiation of differentiation.

### Examples:

The data herein shows that it is possible to generate mammalian haploid stem cell lines from parthenogenetic mouse blastocysts derived from ethanol or strontium activated oocytes. Detailed molecular characterization of the haploid stem cells shows that these cells express all classical markers of diploid ES cells, carry 20 chromosomes, and largely maintain genome integrity. Functionally, these haploid stem cells can differentiate into cells from all three germlayers in vitro and *in vivo*. Although our lines and subclones are stable and in some cases have been maintained for over 70 passages, some haploid cells become diploid. The mutagenesis data suggest that these cells do not become diploid via cell fusion, but rather via failed cytokinesis and/or endoreplication of the genome. Most importantly, the haploid stem cells can be mutated and, in all cases, these mutations are homozygous indicating that such haploid cells can be used to analyse recessive and disease phenotypes in various cell lineages *in vitro*.

These results allow to combine the power of a haploid genome with pluripotency of embryonic stem cells. Recessive genetic screens have elucidated a wide variety of biological processes over the last century and thus markedly contributed to the understanding of normal development, basic physiology, and disease. Reverse genetics is feasible using a vector system that provides immediate confirmation of gene function in the same clones using Cre mediated conversion of the splice acceptor sites. Using this system we have indeed been able to functionally validate our approach using clones with conditional Rarg and Drosha mutations. Moreover, a forward genetic screen was performed for ricin toxicity, one of the most dangerous poisons also being used/investigated as a biological weapon by governments. Ricin also gained notoriety for its potential use as toxin in bioterrorism. This screen in haploid stem cells identified the GPCR Gpr107 as an essential molecule required for ricin induced killing. Since no antitoxins are available for treatment of ricin poisoning, molecular inhibition of Gpr107 (and other molecules identified by our screen) are expected to be useful to alleviate ricin toxicity *in vivo*.

### Example 1: Methods

**Example 1.1: Parthenogenic derivation of haploid stem cells.** C57BL/6x129 F1 female mice were super-ovulated using standard protocols and unfertilized oocytes were flushed and collected. For activation, oocytes were exposed to 5% ethanol or 25 mM SrCl₂ as described (Kaufman et al., 1983; Otaegui et al., 1999). Four hours post activation, viable oocytes were transferred into pseudopregnant 129 females, re-collected on embryonic day (ED) 3.5 and cells were derived according to established embryonic stem derivation protocols (Bryja et al., 2006). Parthenogenetically derived stem cells were initially maintained on feeder layers and subsequently adapted to feeder cell free culture conditions. Stem cell medium consisted of DMEM with 15% FCS (Gibco), supplemented with 2 mM L-Glutamate, 1 mM sodium pyruvate, 100 U penicillin/ml, 0.1 mg streptomycin/ml, 1x non essential aminoacids, 50 mM beta-mercoptoethanol (all Sigma) and ESGRO at 1,000 U/ml (Millipore).

**Example 1.2: Genome coverage analysis and SNP mapping.** Genomic DNA preparations were sheared using a Covaris DNA sonicator, adaptor ligated, and subjected to Illumina sequencing (HiSeq) according to the manufacturers protocol. Reads were mapped using Bowtie (allowing for up to 3 mismatches and requiring that reads map to a single genomic position) and coverage was analysed as reads/50 kb window relative to coverage in the parental strains 129 and C57BL/6, considering only unique genomic coordinates. SNPs were retrieved that differ between C57BL/6 and 129 from Sanger (www.sanger.ac.uk/resources/mouse/genomes/) and mismatches observed during genome mapping of the deep sequencing reads against them were evaluated. Each SNP that was covered by the Solexa reads was assigned to C57BL/6 and 129 according to the majority of reads.

**Example 1.3: Alkaline phosphatase activity, immunohistochemistry, and chromosome spreads.** Alkaline phosphatase activity was detected using VECTOR kit SK-5300. Chromosome spreads were performed following established protocols (Nagy et al., 2008). Immunofluorescence of cultured cells or embryoid body (EB) cultures, neural stem cell, and differentiated neural stem cell cultures was performed after fixation in 4% PFA for 1h, blocking and permeabilization in PBS supplemented with 1% Glycine, 2% BSA, 0.2% Triton, and 5% FCS for 1h. Cells were incubated with the primary antibody o/n at 4°C (anti-Nestin, Abcam 6142, 1:300; anti-Gata4, Santa Cruz, 1:500; anti-Oct3/4, BD Transduction, 1:100; anti-Tuj1, Covance RB-435P, 1:1000; anti-cytokeratin 5, PRP160P-100, Covance; anti GFAP, DAKO, 1:200; anti-Sox2, Cell Signaling, L1D6A2 mouse mAB 1:100; anti-Nanog, Abcam ab80892, 1:100), washed, incubated with fluorescent labeled goat secondary antibodies (Molecular Probes) and visualized using a Zeiss Axioplan2 (neural stem cell differentiation), or Zeiss LSM700 confocal microscope (embryoid bodies). For analyses of *in vivo* differentiation, teratomas were collected, fixed o/n in 4% PFA, paraffin embedded, sectioned, and stained with haematoxylin and eosin (H&E), Alcian blue and nuclear fast red, or processed for immunodetection of Nestin and Cytokeratine 5. Primary Abs were detected using biotinylated secondary antibodies. Ab staining was visualized using streptavidin-HRP and DAB and sections counterstained using hematoxylin. Immunohistochemistry was performed using the Ventana automated system. Images were collected using Zeiss miraxscan. Of note, teratomas were assessed by a certified pathologist.

**Example 1.4: Flow cytometry.** For FACS analyses, cells were trypsinized, washed, and then incubated with 10µg /ml Hoechst33342 while pre-plating for 30 minutes. Subsequently, cells were collected by centrifugation, and FACS sorted for DNA content (as well as FSC-A and SSC-A) using BD FACSAriaIII. Intracellular staining (for FACS subsequent to trypsinization) was performed using the same primary and secondary antibodies as described for immunohistochemistry staining.

**Example 1.5: Gene expression analyses.** RNA was purified using QIAgen RNeasy Mini Kit. Reverse transcription, DNA labeling and microarray hybridization was done according to the manufacturers protocols (Agilent) using the x44K Mouse Genexpression Array DesignID 14868. For qPCR analyses, RNA was purified using QIAgen RNeasy Mini Kit and reverse transcribed using the iScript Kit (Biorad). Amplification was monitored with iQ SYBR Green supermix using the iQ5 Real Time PCR detection System (Biorad). Expression levels were calculated using the ΔΔct method with Gapdh as housekeeping gene. The following PCR primers were used:

| | | |
|---|---|---|
| nanog | GCAAGAACTCTCCTCCATT (SEQ ID NO: 3) | forward |
| | ATGCGTTCACCAGATAGC (SEQ ID NO: 4) | reverse |
| oct-4/pou5f1 | TCACTCACATCGCCAATC (SEQ ID NO: 5) | forward |
| | CCTGTAGCCTCATACTCTTC (SEQ ID NO: 6) | reverse |
| sox2 | CTCGCAGACCTACATGAAC (SEQ ID NO: 7) | forward |
| | CTCGGACTTGACCACAGA (SEQ ID NO: 8) | reverse |
| klf4 | TCTCTCTTCTTCGGACTCC (SEQ ID NO: 9) | forward |
| | CTGGACGCAGTGTCTTCT (SEQ ID NO: 10) | reverse |
| c-myc | GTACCTCGTCCGATTCCA (SEQ ID NO: 11) | forward |
| | CATCTTCTTGCTCTTCTTCAG (SEQ ID NO: 12) | reverse |
| sall4 | AACTTCTCGTCTGCCAGT (SEQ ID NO: 13) | forward |
| | GAGTCATGTAGTGTACCTTCA (SEQ ID NO: 14) | reverse |
| kfl2 | CTCAGCGAGCCTATCTTG (SEQ ID NO: 15) | forward |
| | AGAGGATGAAGTCCAACAC (SEQ ID NO: 16) | reverse |
| gata-4 | GTGAGCCTGTATGTAATGC (SEQ ID NO: 17) | forward |
| | CTGCTGGCGTCTTAGATT (SEQ ID NO: 18) | reverse |
| hand1 | CCTTCAAGGCTGAACTCA (SEQ ID NO: 19) | forward |
| | CGCCCTTTAATCCTCTTCT (SEQ ID NO: 20) | reverse |
| gata-6 | CTCCTACTTCCTCTTCTTCTAA (SEQ ID NO: 21) | forward |
| | CGTCTTGACCTGAATACTTG (SEQ ID NO: 22) | reverse |
| foxa2 | GAGCCGTGAAGATGGAAG (SEQ ID NO: 23) | forward |
| | GTGTTCATGCCATTCATCC (SEQ ID NO: 24) | reverse |
| sox17 | GCCGATGAACGCCTTTA (SEQ ID NO: 25) | forward |
| | CAACGCCTTCCAAGACTT (SEQ ID NO: 26) | reverse |
| krt18 | TTGCCGCCGATGACTT (SEQ ID NO: 27) | forward |
| | CAGCCTTGTGATGTTGGT (SEQ ID NO: 28) | reverse |
| zfp42/rex1 | CTGCCTCCAAGTGTTGTC (SEQ ID NO: 29) | forward |
| | GAACAATGCCTATGACTCAC (SEQ ID NO: 30) | reverse |
| drosha | CCAAGATGATCCAACTCCTT (SEQ ID NO: 31) | forward |
| | GGTGCTGATTCTGAACAATG (SEQ ID NO: 32) | reverse |
| rarg | CACCATTTGAGATGCTGAG (SEQ ID NO: 33) | forward |
| | GGCTTATAGACCCGAGGA (SEQ ID NO: 34) | reverse |

**Example 1.6: Differentiation of stem cells, teratoma formation, and chimeric mice.** For embryoid body (EB) formation, stem cells were trypsinized and cultured in absence of LIF either in hanging drops or in bacterial dishes. For retinoic acid (RA) induced differentiation, cells were grown in presence of 0.1 µM RA for 1 week, plated at density of 1 million per 10 cm dish and assayed 72 hours later. For myoblast differentiation, stem cells were cultured in hanging drops for 4.5 days in stem cell medium in absence of LIF and subsequently rinsed onto gelatinized cell culture dishes. Adhering cell aggregated were fed every 3^{rd} day by replacement of 8ml/10ml stem cell medium without LIF. Movies of beating myoblasts were recorded on days 11-13 at 36 shots/second using a Zeiss Axiovert 200M and a CoolSNAP HQ². Derivation of neuronal stem cells and further differentiation of neuronal stem cells into GFAP⁺ astrocytes and Tuj1⁺ neurons was performed as described (Pollard et al., 2006). For teratoma formation, cells were injected testicular or subcutaneously into nude mice and teratoma growth was monitored. To generate chimeric mice, the diploid fraction of HMSc2 stem cells was purified using flow cytometry, cultivated for 7 days, injected into C57BL/6 ED3.5 blastocysts, and transferred into pseudopregnant 129 females. Percentage chimerism was determined by coat colour.

**Example 1.7: Retroviral infection of stem cells.** Oct4 enhanced gene trap retroviruses carrying a splice acceptor followed by a neomycin resistance gene in 3 reading frames and Oct4 binding sites to enhance transcription (Schnutgen et al., 2008) were packaged in Platinum E cells (Cell Biolabs), concentrated by centrifugation (25,000 rpm, 4°C, 4h) and applied to stem cells with 2µg polybrene per ml for 8 hours. Selection for gene trap insertions was done using G418 (Gibco) at 0.2mg/ml. To estimate numbers of integrations 500.000 cells were plated on 15 cm dishes, selected for integrations using G418 selection and colonies counted after 10 days. For comparison, 5.000 cells were plated without selection.

**Example 1.8: Inverse PCR.** The protocol for inverse PCR was adapted based on Carette et al (Carette et al., 2011). In brief, genomic DNA preparations were digested using DpnII or MseI, purified using the QIAquick Gel Extraction Kit, and fragments ligated at a concentration of 3µg/ml over night. The ligase was then heat inactivated and rings were re-digested in ligase buffer using the enzymes NheI and PvuII. Linearized fragments were purified using the QIAquick Gel Extraction Kit and subjected to PCR using Accuprime Taq polymerase (Invitrogen), primers FS Solexa upstream and FS Solexa downstream, and a BioRad Thermal Cycler. The program of 95°C for 30 sec, 60°C for 30 sec, and 68°C for 105 sec was repeated 36 times. Amplicons were loaded on agarose gels, eluted and subjected to deep sequencing using an Illumina Genome Analyzer and primer FS flowcell. The following iPCR primers were used:
upstream primer
   AATGATACGGCGACCACCGAGATCGCCAGTCCTCCGATTGA (SEQ ID NO: 35)
downstream primer
   CAAGCAGAAGACGGCATACGAGTTCCTATTCCGAAGTTCCTATTCTCTA (SEQ ID NO: 36)
flowcell sequencing primer
   TGATTGACTACCCGTCAGCGGGGGTCTTTCA (SEQ ID NO: 37)

**Example 1.9: Mapping of viral integration sites.** Solexa reads were mapped to the mouse genome using Bowtie and requiring a unique best match to the genome. ENSEMBL gene annotations were used to determine the fraction of integration sites in introns, exons, UTRs, promoters (defined as 2kb upstream of the transcriptional start site), and the remaining intergenic regions. ENSEMBL transcripts were split into 10 bins according to their expression levels in our haploid stem cells as measured by their absolute signal on the Agilent array used for transcriptome analysis and the fraction of transcripts with viral integrations was assessed in each bin. For the equivalent gene-based analysis, the most highly expressed transcript was considered for each gene. The analysis was repeated to estimate the coverage of viral integrations with sub-samples of the total insertions sites.

For confirmation of mapped integration sites the following primers were used:

| | |
|---|---|
| madcam1-F | AGTCTCTCCTTTGCCCTGCTACTGG (SEQ ID NO: 38) |
| madcam1-R | CACAGGCATTGAACAGTTTTGTTGG (SEQ ID NO: 39) |
| drosha-F | TTCGAGTTATAGACTGTAATGAGCC (SEQ ID NO: 40) |
| drosha-R | CCTACACTCTCTAGCAACGGAAGCC (SEQ ID NO: 41) |
| RARG-F | GCTGTTGTCACCCTTGTGCAT AAGCC (SEQ ID NO: 42) |
| RARG-R | AGATGCTGGGAATGGAACCCTGGTCC (SEQ ID NO: 43) |
| Ap4s1-F | GTAGCTTAGAAACTCTGGCCACTGG (SEQ ID NO: 44) |
| Ap4s1-R | CAGTGAAGTCTGAATACAGAGAATGG (SEQ ID NO: 45) |
| Arap1-F | GTCCATGCAGGTTTGAGTGACTCC (SEQ ID NO: 46) |
| Arap1-R | GACCTCCAGCTACAGAGGACAGAGCC (SEQ ID NO: 47) |
| Evx1-F | TGTCAAGGGCAAGAGCTGCGAAGG (SEQ ID NO: 48) |
| Evx1-R | CCAATGTCAAACCGGAAGGGAGAAGG (SEQ ID NO: 49) |
| Bcl211-F | GAGTTACAGATGACTGCGAGCTGCC (SEQ ID NO: 50) |
| Bcl211-R | GAAGCATTGAGTAGCTTTACCTGCC (SEQ ID NO: 51) |
| 2210012G12Rik-F | GTAGACCTGACTTGACTGGCTTGG (SEQ ID NO: 52) |
| 2210012G12Rik-R | GATGCTCATCTTACCAAACGCATCTC (SEQ ID NO: 53) |
| Tit-F | CTTCGACCGTCTGGTCCTCAAGAGG (SEQ ID NO: 54) |
| Tit-R | GAAACCAGCCTGATCTACATAGTGG (SEQ ID NO: 55) |
| chr2:50928851-F | ACTTCCGACAAGAT TCTCAGTCC (SEQ ID NO: 56) |
| chr2:50928851-R | CGTGACCTTTGGGTGTGTAATGCC (SEQ ID NO: 57) |

**Example 1.10: Protein quantification and differentiation in single stem cells.** Differentiation analysis was carried out using a modification of high content screening (HCS) protocols we have previously published analysing the loss of Oct4 expression as a measure of differentiation (Walker et al., 2010; Walker et al., 2007). All HCS experiments were carried out with the feeder free subclone HMSc2-27 at >50 passages. Cells were cultured in parallel in separate rows of 96-well tissue-culture plates (Greiner). Cells were trypsinized to a single-cell suspension and plated at 6000 cells/well into wells that had been pre-coated with a fibronectin/gelatin mixture (12.5 µg/mL fibronectin, Sigma and 0.02% gelatin in water, Millipore). Cells were fixed at 24h, 48h, and 72h time-points with 4% paraformaldehyde for 15 min at room temperature. Immunostaining for Oct4 was carried out as follows: fixed cells were blocked for 30 min at room temperature, permeabilized with 0.1% Triton X-100 / PBS for 1 h at 4C, washed once with permeabilization buffer (PB, 5 % FBS and 0.3% Triton X-100 in PBS), then incubated with mouse monoclonal anti-Oct4 antibody (BD, 1:100) for 1 h at room temperature. Then, cells were washed four times with PBS and incubated with antimouse IgG1 AlexaFluor488 (Invitrogen, 1:100) for 1h in a 1:5000 dilution of DAPI in PB. Plates were then imaged on a ThermoFisher Cellomics ArrayScan VTi automated fluorescence microscope. Data were acquired using a minimum of 9 wells for each time point and condition. An algorithm was designed in the R language based upon nuclear size and DAPI intensity (DNA content) to distinguish haploid cells from diploid cells within mixed cultures of the haploid cell line HMSc2-27. To ensure the fidelity of our haploid vs. diploid analyses, the R algorithm called cells "haploid" only if their DAPI intensity was below the mean DAPI intensity of the haploid controls and called cells "diploid" only if their DAPI intensity was above the mean DAPI intensity of the diploid controls (see Fig. 9).

**Example 1.11: Reversion of the splice acceptor element in the retrovirus.** Clones carrying the gene trap vector were transiently transfected with a plasmid encoding for Cre recombinase as well as GFP, FACS sorted for GFP positive cells, plated at clonal density, picked, expanded and analysed for inversion using PCR analysis.

**Example 1.12: MicroRNA Sensor experiments.** The pSIN-TRE-dsRedmiR30/shRNA-PGK-Venus-shRNA target site vector was used. This vector is a derivative of pSENSOR that enables fluorescence (dsRed)-based monitoring of shRNA expressing cells (Fellmann et al., 2011). Two variants of this vector harboring a potent shRNA targeting Firefly Luciferase (shLuc.1309) with or without its specific target site in the 3'UTR of Venus were each cotransfected with MSCV-rtTA3-PGK-Puro into control wild type ES cells as well as ES cells with antisense (AS) and sense (S) integrations of the mutagenesis vector using Lipofectamine 2000 according to manufacturers protocols. 8 hours after transfection, transfected cells were treated with doxycyline (1 µg/ml) to induce shRNA expression, and after 48 hours, shRNA expressing (dsRed+) cells were analysed for Venus reporter expression level on a FACS-Aria-III flow cytomter (BD).

**Example 1.13: Ricin screen.** Ricin crude extracts in cell culture medium was generated as in (Simmons and Russell, 1985) and concentration was titrated to kill all cells efficiently within 3-4 days. In order to identify genes involved in ricin toxicity, we plated 25 million cells of the mutagenized library described above (Figure 13) in five 15 cm dishes. The library used had a complexity of about 7.5 million different, genetically independent mutations. On a sixth 15 cm plate, 5 million non-mutagenized cells of subclone HMSc2-27 were plated. Cells were maintained in stem cell culture medium in presence of ricin for 2 weeks. At this point, several hundred distinct colonies had appeared on otherwise empty plates while the control plate was completely free of colonies with typical stem cell morphology. To purify ricin insensitive cells further, all cells were trypsinized and replated on an equal surface. Ricin selection in control and library was extended by 1 week. While control plates were entirely free of colonies now, library plates begun to overgrow. We lysed all cells in one pool, purified DNA and subjected the DNA to inverse PCR and deep sequencing (described above) to retrieve all viral integration sites in cells that remained after 3 weeks of constant ricin treatment.

**Example 1.14: Gpr107 knockdown.** A shRNA targeting Gpr107 was designed (TGCTGTTGACAGTGAGCGCAACTAGCTTATTCATAGCCAATAGTGAA-GCCACAGATGTATTGGCTATGAATAAGCTAGTTTTGCCTACTGCCTCGGA, SEQ ID NO: 58) and cloned into LMN (MSCV-miR30-PGK-NeoR-IRES-GFP) as described (Zuber et al., 2010; Zuber et al., 2011). A retroviral vector expressing GPF and shRNA to knock down Gpr107 or a scrambled control shRNA were packaged in Platinum E cells and HMSc2-27 and NIH3T3 cells were transfected in triplicate. After 72 hours, cells were split onto 2 10cm plates, one plate was left untreated and one plate was treated with ricin (1:250 of crude extract for NIH 3T3, 1:2000 of crude extract for HMSc2-27). Analysis of survival was performed after 48 hours following addition of ricin. Cells were analysed by flow cytometry for absolute number, eGFP expression and viability (propidium iodine staining) using an LSR Fortessa (BD).

### Example 2: Derivation of haploid cell lines from parthenogenic murine blastocysts

Parthenogenetic embryos develop from haploid oocytes and thus contain only the maternal genome. However, all reported cell lines derived from parthenogenetic embryos carry a diploid set of chromosomes (Kaufman et al., 1983). It was previously not known that haploid cells might still be present in parthenogenetic early embryos and that haploid stem cells could be derived from such blastocysts. To accomplish this, oocytes from superovulated C57BL/6x129 F1 females were activated by exposure to 5% ethanol or 25 mM SrCl₂. Activated oocytes were then transferred into pseudo-pregnant recipients (Figure 1A). At embryonic day (ED) 3.5, compacted morulae and blastocysts were harvested and cultivated under conditions used to derive stem cells. FACS analysis showed that a small number of the parthenogenetically derived cells indeed displayed a reduced DNA content (Figure 8A). Several rounds of FACS purification of this population and subsequent expansion resulted in two independent cell lines derived from two distinct blastocysts, hereafter termed HMSc1 and HMSc2, with a In chromosome set in the G1 phase and a 2n chromosome set in the G2 phase of the cell cycle (Figure 1B, Figure 8A). Chromosome spreads showed that both cell lines carry a haploid set of 20 chromosomes (Figure 1C, Figure 8B). Of note, both cell lines have now been passaged for > 50 times without any signs of proliferative crisis. Thus, exploiting activation of meiotic oocytes and parthenogenetic derivation of blastocysts allowed to establish mouse cells with a haploid chromosome set.

### Example 3: Genome integrity

To genetically characterize the established haploid cells, their genomes were compared to the genomes of the parental mouse strains C57BL/6 and 129 using deep sequencing and a discriminative coverage analysis was performed. Briefly, the counts of sequencing reads not counting duplicates that map to unique genomic positions in a sliding window of 50kb (10kb offset) were assessed, normalized to the total number of reads that mapped to the genome in each library. As expected, it was found that the strains differed at many positions. Therefore deep sequencing reads from genomic DNA of both the haploid cell lines to both parental strains individually were compared and in a combined fashion in which focus was on deviations with respect to both parental strain (Figure 1D, E). In total, it was found that HMSc1 differed from the parental strains by more than 2 fold (multiple-testing corrected p-value≤10⁻³) in 1553 overlapping windows corresponding to 219 non-overlapping regions, HMSc2 had 568 windows (113 regions) with increased and 61 windows (10 regions) with decreased read-counts (≥2-fold; multiple-testing corrected p-value10-3). Of note, 1155 windows with increased and 99 windows with decreased read-counts overlapped between HMSc1 and HMSc2, while only 397 and 4 were distinct, respectively. Thus, taken together, the haploid cell lines display a limited number of defined CNVs compared to the parent strains, suggesting that they likely harbor small duplications and deletions similar to previous reports using established ES cell (Baker et al., 2007) or iPS cell lines (Hussein et al., 2011).

To confirm that both haploid cell lines are indeed distinct given the similarity in copy number analysis, the fact that they were derived from oocytes of C57BL/6 x 129 intercrosses was used, such that meiotic recombination should result in distinct haplotype structures. The deep sequencing reads to SNPs obtained from the Sanger Institute small nucleotide polymorphisms (SNP) release were compared that differed between both mouse strains. Our sequences covered 1.5 million (HMSc2) and 1.7 million (HMSc1) distinct SNPs and allowed a unique assignment of the corresponding genomic regions to one of both parents. Comparing the SNPs present in HMSc1 versus HMSc2 cells indeed confirmed that two independent haploid clones were derived (Figure 8C). In summary, the CNV analysis together with FACS and chromosome spreads show that both cell lines are derived independently and exclude that HMSc1 and HMSc2 have substantial parts of the genome or individual chromosomes deleted or duplicated.

### Example 4: Expression of prototypic stem cell markers

It was next tested whether the haploid cell lines express prototypic embryonic stem cell markers, i.e. whether the haploid cells are indeed stem cells. Both parthenogenetically derived haploid HMSc1 and HMSc2 lines exhibited typical morphologies of ES cells and stained positive for the ES cell marker alkaline phosphatase (Figure 2A). Immunolabelling to detect Oct4, Nanog, and Sox2 (Figure 2B) confirmed that HMSc1 and HMSc2 cells express prototypic ES cell markers. Transcriptome analysis showed that the expression profile for both haploid cell lines closely resembled that of the diploid ES cell line IB10/C (Figure 9A). To focus on genes with the highest discriminatory value, a set of 100 genes was analysed for maximum difference in gene expression in either direction between mouse embryonic fibroblasts (MEFs) and diploid ES cells, amongst them *Nanog*, *Oct4* and *Klf4* (Chambers et al., 2003). Analysis of this gene set showed that both HMSc1 and HMSc2 cell lines exhibit an expression signature that closely resembles that of the bona fide diploid ES cell line IB10/C (Figure 9B). Quantitative PCR analyses confirmed that both HMSc1 and HMSc2 cells express prototypic ES cell markers (Figure 2C) (Elling et al., 2006; Takahashi and Yamanaka, 2006). Taken together, global transcriptome profiling and expression analysis of prototypic stem cell markers confirmed the stem cell nature of both haploid cell lines.

### Example 5: Differentiation potential of haploid stem cells in vitro

To test the differentiation potential of our haploid ES cells, we first assayed for embryoid body (EB) formation. Both haploid cell lines readily underwent EB formation and EB cells expressed the endodermal marker Gata4 (Figure 2D). Differentiation was further confirmed using real time PCR. The prototypic ES cell markers Nanog, Rex1, Oct4, Sox2, Klf4, Sall4, and Kfl2 (Chambers et al., 2003; Takahashi and Yamanaka, 2006) were downregulated in EBs while mRNA expression of the lineage commitment markers Hand1 (mesoderm, trophectoderm), Nkx2-5 and Brachyury (both mesoderm), Nestin (neural), Gata4, Gata6, Foxa2 (all early endoderm), Sox17 (endoderm and mesoderm), Cxcr4R (endoderm), and Keratin18 (ectoderm) was upregulated relative to the parental haploid ES cells (Figure 2E, Figure 9C). These results indicate that the haploid ES cells are capable of differentiation into several lineages of all three germ-layers.

### Example 6: In vivo differentiation potential

To evaluate the ability of the established haploid ES cell lines to contribute to adult mice, cells from the Agouti⁺ ES line HMSc2 were injected into ED 3.5 blastocysts. To assure competitive growth and thus efficient contribution, diploid cells derived from haploid HMSc2 were used. Coat colour chimerism was observed in 6 animals out of 25 mice born (Figure 3A). To analyse contribution of the entirely maternal derived cells to various organs as previously reported for parthenogenotes (Thomson and Solter, 1988), a distinguishing PCR was performed and detected HMSc2 derived cells in multiple tissues (Figure 10A). To test the intrinsic differentiation potential of our haploid stem cells, in vivo teratoma assays were performed. Similar to diploid ES cell controls, injection of both HMSc1 and HMSc2 cells always resulted in the formation of teratomas within 4-8 weeks.

In teratomas derived from both haploid stem cell lines mesoderm derived muscle cells, endoderm derived alcian blue positive epithelial tissues that produce mucin, neuroectoderm derived Tuj1⁺ neurons, as well as ectoderm-derived Cytokeratin 5⁺ epithelial tissues were observed (Figure 3B). In addition, bona fide cartilage tissue, fat, keratinized multilayered epithelium, pigmented epithelium, sebaceous sweat glands, glandular and neuronal tubules, or ciliated respiratory epithelium were observed (Figure 10B-I). These data show that haploid stem cell derived cells have the potential to contribute to chimeric mice and that they can differentiate in vivo into cells of all three germ layers.

### Example 7: The ability of stable growth and differentiation is intrinsic to haploid stem cells

To assess whether the haploid stem cells have the intrinsic ability for stable growth, several individual cell clones were established by plating single haploid cells directly after FACS purification. These subclones were established in feeder free conditions and derived from both HMSc1 and HMSc2 parental lines that were previously cultured for more than 30 passages. All derived subclones expressed the stem cell markers Oct4 and Sox2 (Figure 4A, Figure 11) and formed EBs that contained Gata4⁺ endodermal cells and Tuj1⁺ neurons (Figure 4A, Figure 12). The haploid subclone HMSc2-27 was chosen for further studies based on its growth rates and numbers of stable haploid cells (Figure 13A, B).

Typical stem cell morphologies, protein expression of Oct4, Nanog, and Sox2, and a haploid set of chromosomes were confirmed for the HMSc2-27 subclone (Figure 13C-F). The growth rates of HMSc2-27 cells at different haploid:diploid seeding ratios were comparable to that of purely diploid HMSc2-27 cells (Figure 4B). Of note, these growth rates are comparable to that of previously established ES cell lines. Kinetic studies on diploid versus haploid cell ratios in cultures of HMSc2-27 cells showed that a large fraction of these cells maintains haploidy for a period of 7 passages (Figure 4C). Differentiation of HMSc2-27 stem cells into EBs followed by lineage specific differentiation protocols showed that these cells have the capability to form Gata4⁺ endoderm, Tuj1⁺ neuronal lineage (Figure 4A), and mesodermal "beating" myoblasts (Figure 4D, for synchronous contractions see Suppl. Movie 1 and 2). Moreover, in in vivo teratoma assays HMSc2-27 cells can differentiate into cells of all germ layers (not shown). To confirm the subcloning experiment, i.e. to make sure that cloning from a single haploid stem cell indeed works, GFP positive subclones were generated. All cells from the established subclones expressed GFP, irrespective if they were at a stage of haploidy or diploidy (Figure 4E). Since diploid cells cannot become haploid, these experiments confirm that all cells of such clones must have been derived from a single haploid cell.

The HMSc2-27 subclone allowed us to examine the differentiation potential of these cells at a haploid versus diploid state. Haploid HMSc2-27 stem cells were indeed able to downregulate Oct4 and form Tuj1⁺ neurons and Gata4⁺ endodermal cells in EB cultures (Figure 4F). It was next attempted to differentiate haploid HMSc27 cells directly (without formation of EBs) into neural and astrocyte lineages (Pollard et al., 2006). HMSc2-27 cells were able to differentiate into neural stem cells (NSCs) as defined by Nestin expression. Moreover, upon further differentiation GFAP⁺ astrocytes and Tuj1⁺ neurons were observed (Figure 5A). It was next tested whether differentiation and a state of haploidy are mutual exclusive, i.e. whether haploid cells need to become diploid before they differentiate. We therefore gated for the lineage markers Oct4 and Nestin in embryonic stem cells, neural stem cells, and differentiated neural stem cell cultures and assayed cells for DNA content. Whereas nearly all Oct4⁺ cells remained haploid under all culture conditions, haploid as well as diploid Nestin⁺ neural progenitor cells were observed. However, when such neural progenitors were further differentiated towards astrocytes and neurons, all Nestin positive cells became diploid within 4 days (Figure 5B).

The differentiation capacity of haploid cells was further assessed using high content screening analysis of Oct4 expression (Walker et al., 2007), DAPI intensity and nuclear area, enabling automated determination of the differentiation state of haploid and diploid HMSc2-27 cells (Figure 14). Stem cells cultured in presence of LIF for 72h maintained high levels of Oct4 as depicted by mean Oct4 expression intensity (Figure 5C, left panel). Distribution analysis showed that HMSc2-27 cells that became diploid expressed a slightly higher level of Oct4 than haploid cells, consistent with the larger nucleus. Upon LIF withdrawal, Oct4 expression substantially decreased in haploid and diploid HMSc2-27 cells (Figure 5C, middle panel). Moreover, differentiation in response to 0.5 µM retinoic acid dramatically reduced Oct4 expression in haploid and diploid HMSc2-27 cells to background levels (Figure 5C, right panel), similar to results obtained using 0.1µM retinoic acid, indicating efficient differentiation. These data show that haploid stem cells can differentiate at similar kinetics as diploid ES cells and, importantly, that haploid stem cells can maintain haploidy even upon initiation of differentiation.

### Example 8: Retroviral mutagenesis

The idea to establish haploid stem cells was to create a tool for forward and reverse genetics at the genomic scale. To demonstrate the power of mutagenesis in haploid mouse stem cells, 5x10⁸ cells of a freshly FACS purified haploid culture of HMSc2-27 were infected with a previously reported retrovirus containing a reversible gene trap (Schnutgen et al., 2008). This vector also contains removable Oct4 binding sites (Schnutgen et al., 2008) which allow for insertions into genes that show minimal or none detectable expression in stem cells. After infection, 7.5x10⁶ independent genomic insertions were generated as estimated from colony formation assays.

Stem cell colonies were then pooled and 10µg of genomic DNA corresponding to 3 million cells were analysed to map the viral insertion sites by inverse PCR and deep sequencing. We could unambiguously identify 176,178 insertions. About half of the insertions were mapped to intergenic regions and ~ 51% of insertions occurred in promoter regions and intragenic regions encompassing 8203 different genes (5'and 3' UTR, 1^{st} intronic, other intronic, and coding regions) (Figure 6A). Among the intragenic insertions, approximately half (53%) were in sense, half in anti-sense direction. Of note, frequent insertions into the 1^{st} intron were observed that most likely will result in complete disruption of gene expression/function. To analyse gene trap efficacy genes were divided into 10 bins based on their expression levels in HMSc2 cells (0-10% equals lowest expression, 90-100% equals highest expressed genes). As expected, more highly expressed genes were more often hit (up to 67%). Importantly, due to the engineered Oct4 binding sites (Schnutgen et al., 2008) we were able to obtain frequent (31%) insertions into genes that show minimal or none detectable expression in stem cells (Figure 6B). We next analysed the numbers of genes that are trapped by all 176,178 insertions or fractions of the total insertions (Figure 6B; all insertions are set to 100% at the X-axis). This analysis shows that mutagenesis has not reached saturation, indicating that higher numbers of insertions will increase the numbers of targeted genes. Considering that the used library consists of 40 x more (7.5x10⁶) independent integrations, the mutagenesis protocol has, in principle, the power to disrupt most genes.

### Example 9: Haploid murine stem cells as a tool for high throughput reverse genetics

Using the retroviral mutagenesis set-up we next picked individual clones, identified the insertion sites of about 1000 cell lines, and selected 10 clones with sense or antisense insertions for further analysis. PCR analysis with site specific primers confirmed that our sequencing approach identified the correct target sites in all 10 cases (Figure 6C). Most importantly these data also show that all 10 clones carry homozygous insertions (Figure 6D), indicating that mutagenesis has occurred in haploid cells and that this approach is indeed feasible for recessive genetics.

Two clones carrying insertions in the genes encoding the *retinoic acid receptor gamma* (*Rarg*) and *Drosha* were functionally validated using parental wild type (Wt) HMSc2-27 cells, or stem cells clones that carry the retroviral vector in antisense orientation. By transient Cre expression we then converted the alleles to sense integrations in which the splice acceptor disrupts gene expression; this approach allows for immediate confirmation of the candidate gene and excludes potential background mutations. Indeed, sense integrations of the splice acceptor results in a near complete absence of Rarg mRNA expression (Figure 6E). Functionally, whereas stem cells carrying the wild type (Wt) allele or the splice acceptors in antisense orientation undergo rapid differentiation and cell death upon retinoic acid treatment, disruption of Rarg expression renders stem cells insensitive to such retinoic acid effect (Figure 6F).

The RNase III Drosha catalyzes the conversion of pri-miRNA transcripts into pre-miRNA stem-loop precursors in the nucleus (Lee et al., 2003). Due to this pivotal role in the initial step of miRNA processing, homozygous *Drosha* inactivation is predicted to severely impair miRNA biogenesis. While a conditional *Drosha* knock-out mouse has been published previously (Chong et al., 2008), no viable *Drosha* knock-out cell line has been reported yet. We were indeed able to generate a *Drosha* mutant stem cell clone following Cre mediated inversion of the splice acceptor (Figure 6G). As reported for ES cells with mutations in the pasha orthologue *Dgcr8* (Wang et al., 2007), which together with Drosha is part of a protein complex called the Microprocessor complex, *drosha* mutant stem cell cannot form cystic embryoid bodies (Figure 6H). To evaluate primary miRNA processing in ES cells harboring the disrupted *Drosha* allele, we monitored the effects of a potent miR30-based shRNA (shmiR.Luc1309) on expression of a transcript harboring a Luc1309 specific shRNA target site (target) in the 3'UTR of a sequence encoding the Venus reporter protein. While expression of shmiR.Luc1309 strongly suppressed Venus expression in normal ES cells, our *Drosha* deficient ES cell clone did not show shRNA-mediated reporter suppression (Figure 6I), indicating a dysfunctional miRNA pathway. These data show that haploid stem cells can be indeed efficiently used for reverse genetics creating reversible and homozygous mutations.

### Example 10: A genome wide screen for genes involved in Ricin toxicity.

Finally, we set out to perform a recessive forward genetic screen at the genome level using the haploid stem cell system. The naturally occurring ricin toxin from the castor oil plant Ricinus communis is highly poisonous. At the molecular level, ricin binds to N-acetyl galactosamine or beta-1,4-linked galactose residues and mannose receptors on the cell surface and ricin molecules are thought to follow retrograde transport via the Golgi apparatus to enter the lumen of the endoplasmic reticulum (ER) where they escape into the cytosol to inactivate ribosomes. Since we found that ricin is highly toxic to mouse ES cells, we used the mutagenized haploid HMSc2-27 cell library and challenged the cells with a lethal dose of ricin. Whereas ricin killed all control stem cells, we observed growth of multiple stem cell colonies from the mutagenized haploid HMSc2-27 cells (Figure 7A). These clones where then pooled and deep sequenced to determine the integration sites. As expected from previous studies, multiple enzymes involved in sugar metabolism were found, i.e. beta-1,4-galactosyltransferase 1 (B4galt1), N-acetyllactosaminide beta-1,6-N-acetylglucosaminyl-transferase (Gcnt2), polypeptide N- acetylgalactosaminyltransferase2 (Galnt2), glycoprotein galactosyltransferase alpha 1,3 (Ggta1), and polypeptide N-acetylgalactosaminyltransferase3 (B4galnt3) (Figure 7B, C). Multiple disruptive mutations in the alpha-(1,3)-fucosyltransferase Fut9 were also obtained and the GDP-fucose transporter1 Slc35c1 was hit (Figure 7B, C). The fucosylation pathway has until now never been associated with ricin toxicity.

Intriguingly, 49 different integrations in the GPCR Gpr107 (LUSTR1) were observed (Figure 7B), suggesting that Gpr107 is a key molecule involved in ricin toxicity. Knockdown of Gpr107 expression in MHSc2-27 cells using shRNA technology in HMSc2 confirmed the central role of this GPCR in ricin toxicity. The key role of Gpr107 in ricin-induced cell death was further confirmed in a different cell type, i.e. NIH 3T3 cells (Fig 7D, E). Of note, our shRNA data are shown as ratios of viable cells recovered from the plates with ricin vs. without ricin treatment (% survival): around 1% of untransfected, control hairpin transfected, or eGFP negative control cells remained still viable after 48 hours of ricin treatment, as compared to their respective non-treated cells; shRNA mediated knockdown of Gpr107 significantly enhanced survival of ricin exposed HMSc2-27 as well as NIH 3T3 cells, but did not fully protect these cells from ricin toxicity. By contrast, genetic ablation of *Gpr107* in haploid HMSc2-27 stem cells as performed for the screen resulted in cells that survived ricin application for 3 weeks and were able to expand and form colonies starting from single, mutated cells. Thus, forward genetic screens are feasible and efficient in our haploid stem cells.

### References

Bryja, V., Bonilla, S., and Arenas, E. (2006). Derivation of mouse embryonic stem cells. Nat Protoc 1, 2082-2087. Carette, J.E., Guimaraes, C.P., Wuethrich, I., Blomen, V.A., Varadarajan, M., Sun, C., Bell, G., Yuan, B., Muellner, M.K., Nijman, S.M., et al. (2011). Global gene disruption in human cells to assign genes to phenotypes by deep sequencing. Nat Biotechnol 29, 542-546. Fellmann, C., Zuber, J., McJunkin, K., Chang, K., Malone, C.D., Dickins, R.A., Xu, Q., Hengartner, M.O., Elledge, S.J., Hannon, G.J., et al. (2011). Functional identification of optimized RNAi triggers using a massively parallel sensor assay. Mol Cell 41, 733-746. Kaufman, M.H., Robertson, E.J., Handyside, A.H., and Evans, M.J. (1983). Establishment of pluripotential cell lines from haploid mouse embryos. J Embryol Exp Morphol 73, 249-261. Nagy, A., Gertsenstein, M., Vintersten, K., and Behringer, R. (2008). Karyotyping mouse cells. CSH Protoc 2008, pdb prot4706. Otaegui, P.J., O'Neill G, T., and Wilmut, I. (1999). Parthenogenetic activation of mouse oocytes by exposure to strontium as a source of cytoplasts for nuclear transfer. Cloning 1, 111-117. Pollard, S.M., Benchoua, A., and Lowell, S. (2006). Neural stem cells, neurons, and glia. Methods Enzymol 418, 151-169. Schnutgen, F., Hansen, J., De-Zolt, S., Horn, C., Lutz, M., Floss, T., Wurst, W., Noppinger, P.R., and von Melchner, H. (2008). Enhanced gene trapping in mouse embryonic stem cells. Nucleic Acids Res 36, e133. Simmons, B.M., and Russell, J.H. (1985). A single affinity column step method for the purification of ricin toxin from castor beans (Ricinus communis). Anal Biochem 146, 206-210. Walker, E., Chang, W.Y., Hunkapiller, J., Cagney, G., Garcha, K., Torchia, J., Krogan, N.J., Reiter, J.F., and Stanford, W.L. (2010). Polycomb-like 2 associates with PRC2 and regulates transcriptional networks during mouse embryonic stem cell self-renewal and differentiation. Cell Stem Cell 6, 153-166. Walker, E., Ohishi, M., Davey, R.E., Zhang, W., Cassar, P.A., Tanaka, T.S., Der, S.D., Morris, Q., Hughes, T.R., Zandstra, P.W., et al. (2007). Prediction and testing of novel transcriptional networks regulating embryonic stem cell self-renewal and commitment. Cell Stem Cell 1, 71-86. Zuber, J., McJunkin, K., Fellmann, C., Dow, L.E., Taylor, M.J., Hannon, G.J., and Lowe, S.W. (2010). Toolkit for evaluating genes required for proliferation and survival using tetracycline-regulated RNAi. Nat Biotechnol 29, 79-83. Zuber, J., Shi, J., Wang, E., Rappaport, A.R., Herrmann, H., Sison, E.A., Magoon, D., Qi, J., Blatt, K., Wunderlich, M., et al. (2011). RNAi screen identifies Brd4 as a therapeutic target in acute myeloid leukaemia. Nature. Elling, U., Klasen, C., Eisenberger, T., Anlag, K., and Treier, M. (2006). Murine inner cell mass-derived lineages depend on Sall4 function. Proc Natl Acad Sci U S A 103, 16319-16324. Takahashi, K., and Yamanaka, S. (2006). Induction of pluripotent stem cells from mouse embryonic and adult fibroblast cultures by defined factors. Cell 126, 663-676. Chambers, I., Colby, D., Robertson, M., Nichols, J., Lee, S., Tweedie, S., and Smith, A. (2003). Functional expression cloning of Nanog, a pluripotency sustaining factor in embryonic stem cells. Cell 113, 643-655. Thomson, J.A., and Solter, D. (1988). The developmental fate of androgenetic, parthenogenetic, and gynogenetic cells in chimeric gastrulating mouse embryos. Genes Dev 2, 1344-1351. Pollard, S.M., Benchoua, A., and Lowell, S. (2006). Neural stem cells, neurons, and glia. Methods Enzymol 418, 151-169. Chong, M.M., Rasmussen, J.P., Rudensky, A.Y., and Littman, D.R. (2008). The RNAseIII enzyme Drosha is critical in T cells for preventing lethal inflammatory disease. J Exp Med 205, 2005-2017. Wang, Y., Medvid, R., Melton, C., Jaenisch, R., and Blelloch, R. (2007). DGCR8 is essential for microRNA biogenesis and silencing of embryonic stem cell self-renewal. Nat Genet 39, 380-385. WO 2008/33469 A1 WO 2001/32015 A1

## Claims

1. A method of generating a mammal cell line of haploid cells comprising obtaining a plurality of haploid cells in an aggregated embryonic stage of development, preferably a blastocyst stage, isolating cells of said plurality, expanding isolated cells of said plurality, selecting and isolating one or more single cells with a haploid genome from said expanded cells, thereby obtaining a cell of a cell line of stably haploid cells.

2. The method of claim 1 further comprising the steps of obtaining a haploid parthenogenic oozyte or embryonic cell of said mammal, transferring said oozyte or embryonic cell into a pseudopregnant female, allowing said oozyte or embryonic cell to grow to a multicellular stage, optionally culturing cell of said multicellular stage to a blastocyst stage, thereby providing said plurality of haploid cells in an aggregated embryonic stage of development.

3. The method of claim 1 or 2 further comprising culturing said cells of the cell culture and isolating haploid cells from said cell culture after up to 50 passages and expanding said isolated haploid cells as new continued cell culture.

4. The method of any one of claims 1 to 3 wherein the parental cell is heterozygous or homozygous, preferably heterozygous.

5. A cell culture of cells with a haploid genome obtainable by a method of any one of claims 1 to 4.

6. Method for mutation analysis comprising providing a cell with a haploid genome, preferably a mammal cell, comprising introducing a mutation into a genetic locus of interest in said cell and observing modified activity of the cell related to said genetic locus.

7. Method according to claim 6, wherein the cell is stable in haploid form for at least 10 passages, preferably a cell obtainable or obtained by a method according to any one of claim 1 to 4.

8. A cell of a cell culture according to claim 5, wherein one or more genes of interest have been inactivated.

9. A haploid mammal cell comprising a knock-out of the gene Drosha and/or Rarg, preferably wherein said cell is of a cell culture according to claim 5.

10. Method of generating cells comprising a phenotype of interest, comprising randomly mutating a plurality of cells with a haploid genome, preferably mammal cells, especially preferred cells obtainable or obtained by a method according to any one of claim 1 to 4, and selecting cells having the phenotype of interest.

11. Method of claim 10, wherein the phenotype of interest is or cell survival or cell growth when said cells are contacted with a toxin or growth inhibitor.

12. Method of claim 11, where said toxin is ricin.

13. Method of any one of claims 10 to 12, wherein the plurality of cells comprises at least 1000 cells.

14. A haploid cell having a resistance to a toxin, preferably ricin, obtainable by a method of any one of claims 8 to 13.

15. A method of screening a cell for genetic targets having activity in toxin resistance comprising generating cells with a toxin resistance according to any one of claims 11 to 13 further comprising identifying a mutation in said cells having toxin resistance as compared to cells without said random mutation.

16. A method of identifying a therapeutic agent against a toxin, comprising identifying a genetic target according to claim 15 and contacting a therapeutic candidate molecule with said genetic target or the gene product of said target, preferably in an isolated cell, and identifying binding events of the candidate with the genetic target or gene product or modified resistance of the cell to the toxin.

17. The method of claim 16, wherein the toxin is ricin and preferably the genetic target is selected from Gpr107, Fut9, Tcf711, Slc35c1, Fgfr2, Galnt2, Mid1, B4galt1, B4galnt3, Plcd3, Ror2, Samd4b, Gcnt2 or Ggta1.

18. The method of treating ricin poisoning in a subject or inducing a ricin resistance in a subject comprising inhibiting any one of Gpr107, Fut9, Tcf711, Slc35c1, Fgfr2, Galnt2, Mid1, B4galt1, B4galnt3, Plcd3, Ror2, Samd4b, Gcnt2 or Ggta1 to said subject, preferably wherein inhibiting is by administration of an inhibitor, especially an antibody specific for any one of Gpr107, Fut9, Tcf711, Slc35c1, Fgfr2, Galnt2, Mid1, B4galt1, B4galnt3, Plcd3, Ror2, Samd4b, Gcnt2 or Ggta1 or an inhibitory nucleic acid, especially comprising or encoding a siRNA or shRNA, especially preferred an inhibitory nucleic acid that reduces mRNA of said genes by at least 20%, especially by at least 50%, in a cell.

19. A modified cell with increased resistance to ricin comprising a reduced expression in any one of Gpr107, Fut9, Tcf711, Slc35c1, Fgfr2, Galnt2, Mid1, B4galt1, B4galnt3, Plcd3, Ror2, Samd4b, Gcnt2, Ggta1, or combinations thereof, as compared to an unmodified cell without increased resistance to ricin.


SEQUENCE LISTING

<110>  IMBA - Institut für Molekulare Biotechnologie GmbH

<120>  haploid cells

<130>  r57519

<160>  58

<170>  PatentIn version 3.3

<210>  1
<211>  27
<212>  DNA
<213>  Artificial

<220>
<223>  primer

<400>  1
gaatgtgagc gcacagggtg atgtgcc                                          27


<210>  2
<211>  25
<212>  DNA
<213>  Artificial

<220>
<223>  primer

<400>  2
cccacagaac acagtcacag ggtcc                                            25


<210>  3
<211>  19
<212>  DNA
<213>  Artificial

<220>
<223>  primer

<400>  3
gcaagaactc tcctccatt                                                   19


<210>  4
<211>  18
<212>  DNA
<213>  Artificial

<220>
<223>  primer

<400>  4
atgcgttcac cagatagc                                                    18


<210>  5
<211>  18
<212>  DNA
<213>  Artificial

<220>
<223>  primer

<400>  5
tcactcacat cgccaatc                                                    18


<210>  6
<211>  20
<212>  DNA
<213>  Artificial

<220>
<223>  primer

<400>  6
cctgtagcct catactcttc                                                  20


<210>  7
<211>  19
<212>  DNA
<213>  Artificial

<220>
<223>  primer

<400>  7
ctcgcagacc tacatgaac                                                   19


<210>  8
<211>  18
<212>  DNA
<213>  Artificial

<220>
<223>  primer

<400>  8
ctcggacttg accacaga                                                    18


<210>  9
<211>  19
<212>  DNA
<213>  Artificial

<220>
<223>  primer

<400>  9
tctctcttct tcggactcc                                                   19


<210>  10
<211>  18
<212>  DNA
<213>  Artificial

<220>
<223>  primer

<400>  10
ctggacgcag tgtcttct                                                    18

<210>  11
<211>  18
<212>  DNA
<213>  Artificial

<220>
<223>  primer

<400>  11
gtacctcgtc cgattcca                                                    18


<210>  12
<211>  21
<212>  DNA
<213>  Artificial

<220>
<223>  primer

<400>  12
catcttcttg ctcttcttca g                                                21


<210>  13
<211>  18
<212>  DNA
<213>  Artificial

<220>
<223>  primer

<400>  13
aacttctcgt ctgccagt                                                    18


<210>  14
<211>  21
<212>  DNA
<213>  Artificial

<220>
<223>  primer

<400>  14
gagtcatgta gtgtaccttc a                                                21


<210>  15
<211>  18
<212>  DNA
<213>  Artificial

<220>
<223>  primer

<400>  15
ctcagcgagc ctatcttg                                                    18


<210>  16
<211>  19
<212>  DNA

<213>  Artificial

<220>
<223>  primer

<400>  16
agaggatgaa gtccaacac                                                    19


<210>  17
<211>  19
<212>  DNA
<213>  Artificial

<220>
<223>  primer

<400>  17
gtgagcctgt atgtaatgc                                                    19


<210>  18
<211>  18
<212>  DNA
<213>  Artificial

<220>
<223>  primer

<400>  18
ctgctggcgt cttagatt                                                     18


<210>  19
<211>  18
<212>  DNA
<213>  Artificial

<220>
<223>  primer

<400>  19
ccttcaaggc tgaactca                                                     18


<210>  20
<211>  19
<212>  DNA
<213>  Artificial

<220>
<223>  primer

<400>  20
cgccctttaa tcctcttct                                                    19


<210>  21
<211>  22
<212>  DNA
<213>  Artificial

<220>
<223>  primer

<400>  21
ctcctacttc ctcttcttct aa                                                22


<210>  22
<211>  20
<212>  DNA
<213>  Artificial

<220>
<223>  primer

<400>  22
cgtcttgacc tgaatacttg                                                   20


<210>  23
<211>  18
<212>  DNA
<213>  Artificial

<220>
<223>  primer

<400>  23
gagccgtgaa gatggaag                                                     18


<210>  24
<211>  19
<212>  DNA
<213>  Artificial

<220>
<223>  primer

<400>  24
gtgttcatgc cattcatcc                                                    19


<210>  25
<211>  17
<212>  DNA
<213>  Artificial

<220>
<223>  primer

<400>  25
gccgatgaac gccttta                                                      17


<210>  26
<211>  18
<212>  DNA
<213>  Artificial

<220>
<223>  primer

<400>  26
caacgccttc caagactt                                                     18


<210>  27

<211>  16
<212>  DNA
<213>  Artificial

<220>
<223>  primer

<400>  27
ttgccgccga tgactt                                                       16


<210>  28
<211>  18
<212>  DNA
<213>  Artificial

<220>
<223>  primer

<400>  28
cagccttgtg atgttggt                                                     18


<210>  29
<211>  18
<212>  DNA
<213>  Artificial

<220>
<223>  primer

<400>  29
ctgcctccaa gtgttgtc                                                     18


<210>  30
<211>  20
<212>  DNA
<213>  Artificial

<220>
<223>  primer

<400>  30
gaacaatgcc tatgactcac                                                   20


<210>  31
<211>  20
<212>  DNA
<213>  Artificial

<220>
<223>  primer

<400>  31
ccaagatgat ccaactcctt                                                   20


<210>  32
<211>  20
<212>  DNA
<213>  Artificial

<220>

<223>  primer

<400>  32
ggtgctgatt ctgaacaatg                                                   20


<210>  33
<211>  19
<212>  DNA
<213>  Artificial

<220>
<223>  primer

<400>  33
caccatttga gatgctgag                                                    19


<210>  34
<211>  18
<212>  DNA
<213>  Artificial

<220>
<223>  primer

<400>  34
ggcttataga cccgagga                                                     18


<210>  35
<211>  41
<212>  DNA
<213>  Artificial

<220>
<223>  primer

<400>  35
aatgatacgg cgaccaccga gatcgccagt cctccgattg a                           41


<210>  36
<211>  49
<212>  DNA
<213>  Artificial

<220>
<223>  primer

<400>  36
caagcagaag acggcatacg agttcctatt ccgaagttcc tattctcta                   49


<210>  37
<211>  31
<212>  DNA
<213>  Artificial

<220>
<223>  primer

<400>  37
tgattgacta cccgtcagcg ggggtctttc a                                      31

<210>  38
<211>  25
<212>  DNA
<213>  Artificial

<220>
<223>  primer

<400>  38
agtctctcct ttgccctgct actgg                                          25


<210>  39
<211>  25
<212>  DNA
<213>  Artificial

<220>
<223>  primer

<400>  39
cacaggcatt gaacagtttt gttgg                                          25


<210>  40
<211>  25
<212>  DNA
<213>  Artificial

<220>
<223>  primer

<400>  40
ttcgagttat agactgtaat gagcc                                          25


<210>  41
<211>  25
<212>  DNA
<213>  Artificial

<220>
<223>  primer

<400>  41
cctacactct ctagcaacgg aagcc                                          25


<210>  42
<211>  26
<212>  DNA
<213>  Artificial

<220>
<223>  primer

<400>  42
gctgttgtca cccttgtgca taagcc                                         26


<210>  43
<211>  26
<212>  DNA
<213>  Artificial

<220>
<223>  primer

<400>  43
agatgctggg aatggaaccc tggtcc                                          26


<210>  44
<211>  25
<212>  DNA
<213>  Artificial

<220>
<223>  primer

<400>  44
gtagcttaga aactctggcc actgg                                           25


<210>  45
<211>  26
<212>  DNA
<213>  Artificial

<220>
<223>  primer

<400>  45
cagtgaagtc tgaatacaga gaatgg                                          26


<210>  46
<211>  24
<212>  DNA
<213>  Artificial

<220>
<223>  primer

<400>  46
gtccatgcag gtttgagtga ctcc                                            24


<210>  47
<211>  26
<212>  DNA
<213>  Artificial

<220>
<223>  primer

<400>  47
gacctccagc tacagaggac agagcc                                          26


<210>  48
<211>  24
<212>  DNA
<213>  Artificial

<220>
<223>  primer

<400>  48

tgtcaagggc aagagctgcg aagg                                          24

<210>  49
<211>  26
<212>  DNA
<213>  Artificial

<220>
<223>  primer

<400>  49
ccaatgtcaa accggaaggg agaagg                                        26

<210>  50
<211>  25
<212>  DNA
<213>  Artificial

<220>
<223>  primer

<400>  50
gagttacaga tgactgcgag ctgcc                                         25

<210>  51
<211>  25
<212>  DNA
<213>  Artificial

<220>
<223>  primer

<400>  51
gaagcattga gtagctttac ctgcc                                         25

<210>  52
<211>  24
<212>  DNA
<213>  Artificial

<220>
<223>  primer

<400>  52
gtagacctga cttgactggc ttgg                                          24

<210>  53
<211>  26
<212>  DNA
<213>  Artificial

<220>
<223>  primer

<400>  53
gatgctcatc ttaccaaacg catctc                                        26

<210>  54
<211>  25

<212>  DNA
<213>  Artificial

<220>
<223>  primer

<400>  54
cttcgaccgt ctggtcctca agagg                                          25


<210>  55
<211>  25
<212>  DNA
<213>  Artificial

<220>
<223>  primer

<400>  55
gaaaccagcc tgatctacat agtgg                                          25


<210>  56
<211>  23
<212>  DNA
<213>  Artificial

<220>
<223>  primer

<400>  56
acttccgaca agattctcag tcc                                            23


<210>  57
<211>  24
<212>  DNA
<213>  Artificial

<220>
<223>  primer

<400>  57
cgtgaccttt gggtgtgtaa tgcc                                           24


<210>  58
<211>  97
<212>  DNA
<213>  Artificial

<220>
<223>  primer

<400>  58
tgctgttgac agtgagcgca actagcttat tcatagccaa tagtgaagcc acagatgtat      60

tggctatgaa taagctagtt ttgcctactg cctcgga                             97